# EUROPEAN PATENT SPECIFICATION

(11) **EP 3 164 169 B1**
(45) Date of publication and mention of the grant of the patent: **07.05.2025**
(21) Application number: 15814436.0
(22) Date of filing: 01.07.2015
(51) Int. Cl.: A61M 5/00, A61M 5/32, A61M 25/00, A61M 25/02, A61M 5/158

(54) **ANTIMICROBIAL/HAEMOSTATIC INTERFACE PAD FOR PLACEMENT BETWEEN PERCUTANEOUSLY PLACED MEDICAL DEVICE AND PATIENT SKIN**
ANTIMIKROBIELLER/HÄMOSTATISCHER SCHNITTSTELLENPFAD ZUR PLATZIERUNG ZWISCHEN PERKUTAN PLATZIERTEN MEDIZINISCHEN VORRICHTUNG UND DER HAUT EINES PATIENTEN
TAMPON D'INTERFACE HÉMOSTATIQUE/ANTIMICROBIEN DESTINÉ À ÊTRE PLACÉ ENTRE UN DISPOSITIF MÉDICAL PLACÉ PAR VOIE PERCUTANÉE ET LA PEAU D'UN PATIENT

(30) Priority: 01.07.2014 US 201462019807 P
(43) Date of publication of application: 10.05.2017
(73) Proprietor: C. R. Bard, Inc., Franklin Lakes, NJ 07417 (US)
(72) Inventor: HOWELL, Glade Harold, Draper, UT 84020 (US); ELANGOVAN, Anthony S., Herriman, UT 84096 (US); BOWN, Matthew W., West Bountiful, UT 84087 (US); VANDERSTEK, Bradley J., West Bountiful, UT 84087 (US); CORUM, Lindsey E., Cottonwood Heights, UT 84121 (US); BEAL, James D., South Jordan, UT 84095 (US)
(74) Representative: Hoffmann Eitle
(86) International application number: PCT/US2015/038853
(87) International publication number: WO 2016/004217

(56) References cited:
- EP-A1- 2 436 421
- WO-A1-2012/132774
- WO-A1-2012/132774
- US-A- 4 755 173
- US-A- 4 775 369
- US-A- 5 342 319
- US-A- 5 342 319
- US-A- 5 342 319
- US-A- 5 686 096
- US-A- 5 722 959
- US-A- 5 885 254
- US-A1- 2004 049 159
- US-A1- 2004 049 159
- US-A1- 2010 076 362
- US-A1- 2010 100 049
- US-A1- 2010 179 473
- US-A1- 2011 009 831
- US-A1- 2011 111 012
- US-A1- 2011 301 619
- US-A1- 2012 065 587
- US-A1- 2012 065 587
- US-A1- 2013 310 764

## Description

### BACKGROUND ART

US 2012/0065587 A1 discloses a safety needle assembly of an infusion set for infusing fluids into a subcutaneously implanted access port, wherein the needle assembly is configured to prevent fluid/vapour escape therefrom so as to reduce or prevent fluid exposure to a clinician using the needle assembly.

US 5342319 A1 discloses a transdermal injection appliance having a pad impregnated with a disinfectant solution such as alcohol, a body with adhesive for holding the pad against the skin of a patient, and a sleeve with a guide passage through the body for guiding an injection needle.

US2004049159 discloses a safety shield apparatus which includes a needle having a distal portion defining a longitudinal axis which is angularly displaced relative to a longitudinal axis defined by a proximal portion of the needle. A shield is mounted with the needle and extensible, via a tubular needle guide movable along the needle, between a retracted position and an extended position. The apparatus may include a needle hub configured to support the proximal portion of the needle. The needle hub can include an appendage which may have at least one opening to facilitate manipulation thereof. A distal end of the shield can be attached to a planar contact surface.

### BRIEF SUMMARY

Briefly summarized, embodiments of the present invention are directed to a safety needle assembly of an infusion set for infusing fluids into a subcutaneously implanted access port. The needle assembly is configured to prevent fluid escape therefrom so as to reduce or prevent fluid exposure to a clinician using the needle assembly.

The invention is defined in claims 1 and 11: It defines a needle assembly (810, 910), comprising: A needle hub; and a needle extending from the needle hub; and an interface pad attached to the needle hub before use of the needle assembly, the interface pad including at least one of a haemostatic agent and an antimicrobial agent, the interface pad configured to be interposed between the needle hub and the patient's skin when the needle is percutaneously inserted into the patient, wherein the interface pad includes shape that matches a shape of a bottom surface of the needle hub, the interface pad including a hole through which the needle is configured to extend. The invention also defines a method of manufacturing a needle assembly , the method comprising: providing a needle hub, a needle distally extending from the needle hub; and permanently adhering an interface pad to a bottom surface of the needle hub, the needle configured to extend through the interface pad, the interface pad including at least one of a haemostatic agent and an antimicrobial agent.

These and other features of embodiments of the present invention will become more fully apparent from the following description and appended claims, or may be learned by the practice of embodiments of the invention as set forth hereinafter.

### BRIEF DESCRIPTION OF THE DRAWINGS

A more particular description of the present disclosure will be rendered by reference to specific embodiments thereof that are illustrated in the appended drawings. It is appreciated that these drawings depict only typical embodiments of the invention and are therefore not to be considered limiting of its scope. Example embodiments of the invention will be described and explained with additional specificity and detail through the use of the accompanying drawings in which:
FIG. 1 is a perspective view of an infusion set including a safety needle assembly according to one example not forming part of the invention;
FIG. 2 is a side view of the needle assembly of FIG. 1;
FIGS. 3A-3C show actuation of needle assembly of FIG. 1;
FIG. 4 shows a perspective view of an infusion set including a safety needle assembly in a first configuration according to one example not forming part of the invention;
FIG. 5 shows a perspective view of the infusion set of FIG. 4 with the safety needle assembly in a second configuration;
FIGS. 6A-6C show actuation of the safety needle assembly of FIGS. 4 and 5
FIGS. 7A-7C shows actuation of a safety needle assembly according to another example not forming part of the invention;
FIGS. 8A and 8B show a bottom view of a safety needle assembly including a fluid isolation component according to one example not forming part of the invention;
FIGS. 9A and 9B are cross sectional side views of a safety needle assembly including a fluid isolation component according to one example not forming part of the invention;
FIG. 10 is a cross sectional side view of a safety needle assembly including a fluid isolation component according to one example not forming part of the invention;
FIG. 11 is a perspective view of a safety needle assembly according to one example not forming part of the invention;
FIGS. 12A and 12B are bottom views of the safety needle assembly of FIG. 11;
FIG. 13 is a top view of a shutter of the safety needle assembly of FIG. 10, including a fluid isolation component according to one example not forming part of the invention;
FIG. 14 is a perspective view of the shutter of FIG. 13 including the fluid isolation component;
FIG. 15 is a cross sectional side view of a safety needle assembly according to one example not forming part of the invention;
FIGS. 16A and 16B are various views of a safety needle assembly according to one example not forming part of the invention;
FIG. 17 is a cross sectional side view of a luer connector including a fluid isolation component according to one example not forming part of the invention;
FIGS. 18A-18B are cross sectional side views of the luer connector of FIG. 17 during use;
FIGS. 19A and 19B show various views of a fluid isolation component together with an infusion set, according to one example not forming part of the invention;
FIG. 20 is a bottom view of a safety needle assembly including a fluid isolation component according to one example not forming part of the invention;
FIGS. 21A and 21B are various views of a needle assembly including a self-sealing pad according to one example not forming part of the invention;
FIGS. 22A and 22B are various views of the self-sealing pad of FIGS. 21A and 21B;
FIGS. 23A and 23B are various views of the needle assembly of FIGS. 21A and 21B;
FIG. 24 is a perspective view of a self-sealing pad according to one example not forming part of the invention;
FIG. 25 is a perspective view of a plurality of catheters including an interface pad according to one example not forming part of the invention;
FIGS. 26A and 26B are side views of a catheter including an interface pad according to one example not forming part of the invention;
FIGS. 27A and 27B are various views of an interface pad according to one example not forming part of the invention;
FIGS. 28A and 28B are various views of an interface pad according to one example not forming part of the invention;
FIGS. 29A and 29B are various views of an interface pad according to one example not forming part of the invention;
FIG. 30 is a side view of a catheter including an interface pad according to one example not forming part of the invention;
FIG. 31 is a side view of a catheter including an interface pad according to one example not forming part of the invention;
FIG. 32 is a side view of a strip of antimicrobial foam for use as an interface pad according to one example not forming part of the invention;
FIG. 33 is a side view of a catheter including an interface pad according to one example not forming part of the invention;
FIG. 34 is a side view of a catheter including an interface pad according to one example not forming part of the invention;
FIG. 35 is a cross sectional side view of the catheter and interface pad of FIG. 34;
FIGS. 36A and 36B are various views depicting one method for forming an interface pad according to one example not forming part of the invention;
FIG. 37 is a side view of a catheter including an antimicrobial interface according to one embodiment;
FIG. 38 is a perspective view of an infusion set including an interface pad according to the invention;
FIGS. 39A-39D are various views of portions of the infusion set of FIG. 38;
FIGS. 40A-40E are various views of an infusion set including an interface pad according to a second aspect of the invention; and
FIG. 41 is a partial cross-sectional side view of the infusion set of FIGS. 40A-40E operably connected to an implanted access port according to one embodiment.

### DETAILED DESCRIPTION OF SELECTED EMBODIMENTS

Reference will now be made to figures wherein like structures will be provided with like reference designations. It is understood that the drawings are diagrammatic and schematic representations of exemplary embodiments of the present invention, and are neither limiting nor necessarily drawn to scale.

For clarity it is to be understood that the word "proximal" refers to a direction relatively closer to a clinician using the device to be described herein, while the word "distal" refers to a direction relatively further from the clinician. For example, the end of a needle placed within the body of a patient is considered a distal end of the needle, while the needle end remaining outside the body is a proximal end of the needle. Also, the words "including," "has," and "having," as used herein, including the claims, shall have the same meaning as the word "comprising."

Embodiments of the present invention are generally directed to a safety infusion set and accompanying needle assembly for infusing fluids, such as chemotherapy agents or other medicaments for example, into an access port or other medical device subcutaneously implanted into the body of a patient. The infusion set and/or needle assembly includes one or more components for isolation of the fluid, including vapors thereof, which may otherwise leak from a needle or other portion of the infusion set. This in turn reduces or prevents possible clinician exposure to the fluid/vapors, which in some cases may be hazardous. Potential harm to the clinician is therefore reduced.

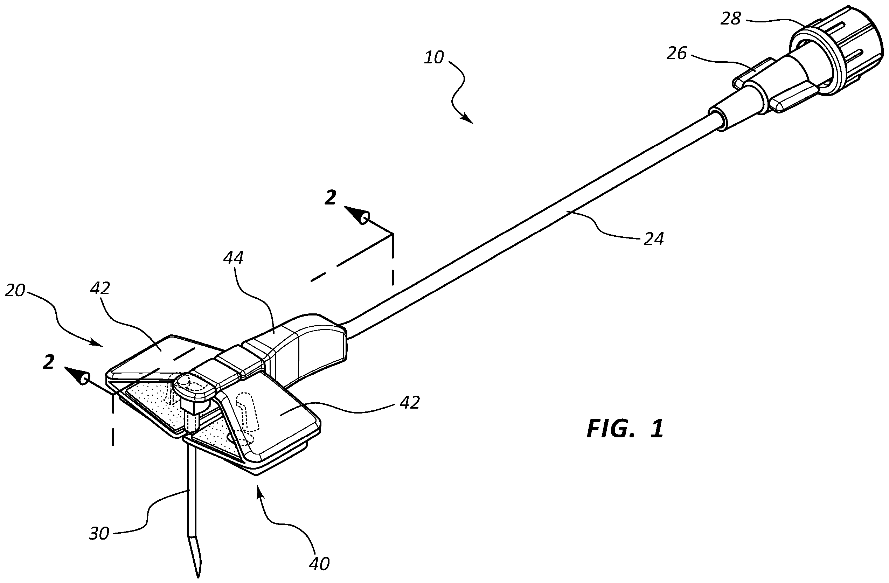

Reference is first made to FIG. 1, which depicts an infusion set generally designated at 10, including a safety needle assembly ("needle assembly") 20 and one or more extension legs 24. The infusion set 10 is employed to gain access to a subcutaneously implanted access port or other device disposed below the skin of a patient in order to infuse medicaments or other fluids into the patient, and to withdraw fluids therefrom. A luer connector 26 is included on a proximal end of the extension leg 24 so as to enable the infusion set 10 to be placed into fluid communication with a fluid delivery device or system. A cap 28 can be disposed in the luer connector 26 to cover the opening thereof.

FIG. 1 shows that the needle assembly 20 includes a needle 30 extending from a handle 44 and in fluid communication with the tubing of the extension leg 24. A needle safety component 40 is also included in the needle assembly 20, including dual extensible wings that are hinged so as to be selectively extended to substantially cover the length of the needle 30 and isolate a distal end 30A thereof after use of the needle assembly 20 in order to prevent an unintended needle stick of the clinician by the needle tip. Examples of such a hinged safety assembly can be found in U.S. Patent No. 5,951,522.

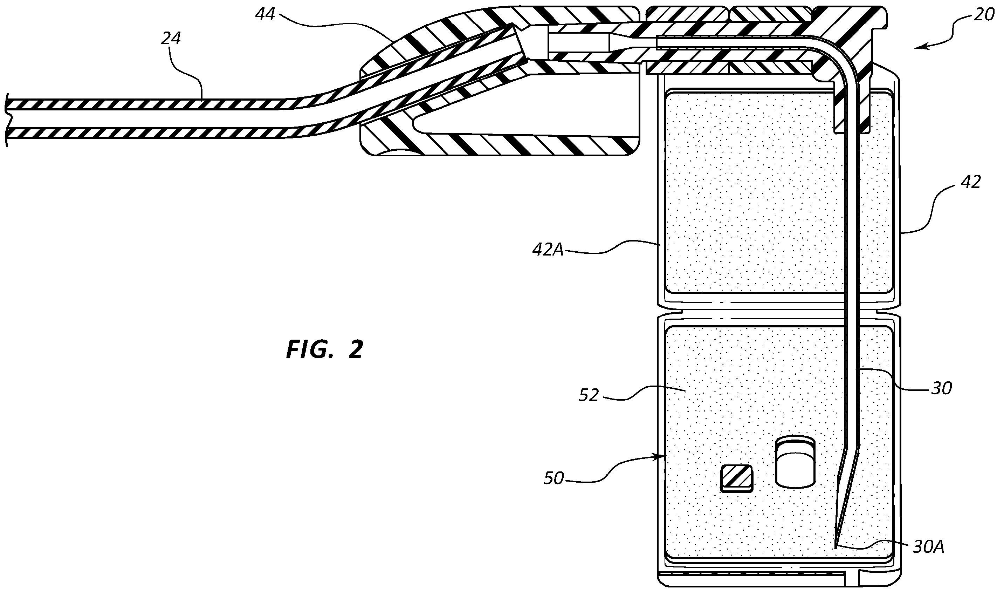

As best seen in FIG. 2, the needle assembly 20 further includes a fluid isolation component 50 for isolating any fluid or vapor that may unintentionally escape from the needle 30 during use of the needle assembly. Specifically, the fluid isolation component 50 in the present example includes absorbent pads 52 disposed on an inner surface 42A of each wing 42 of the needle safety component 40. The pads 52 are disposed such that when the wings 42 of the needle safety component 40 are deployed to cover the distal tip 30A of the needle 30 (FIG. 2), the pads sandwich the body and distal tip of the needle therebetween. Any fluid present on an external surface of the needle or any fluid/vapor leaking from the distal end thereof is captured and absorbed by the pads 52, thus preventing escape of the fluid, which as mentioned above may contain hazardous substances. This in turn protects the clinician from fluid exposure.

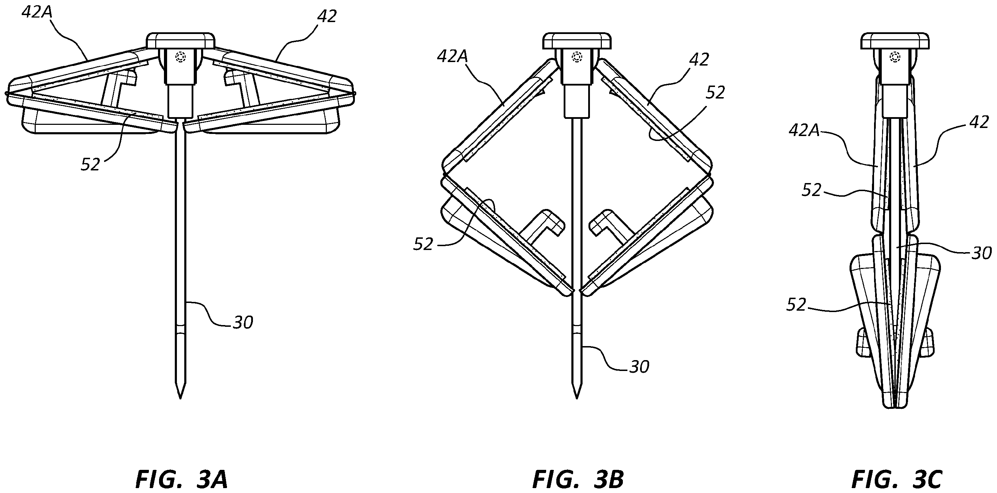

FIGS. 3A-3C show the manner in which the wings 42 of the needle safety component 40 extend to cover the needle 30 and its distal tip 30A, and additionally the manner in which the pads 52 sandwich and partially encapsulate the needle 30, including its external surfaces and its distal tip 30A, to prevent fluid/vapor escape. In one example, the pads 52 can include an absorbent foam and/or rubber material, though many other suitable materials can be employed, including activated charcoal, etc.

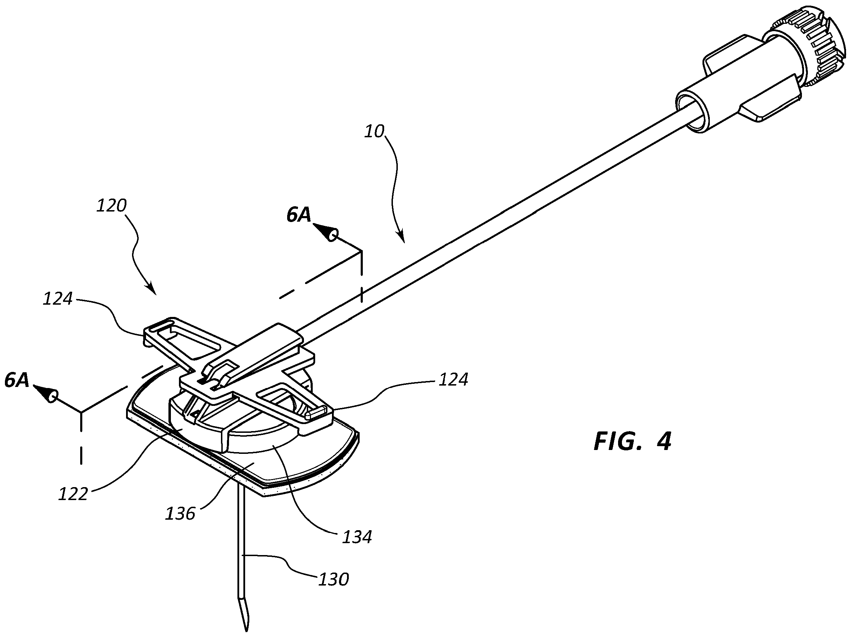

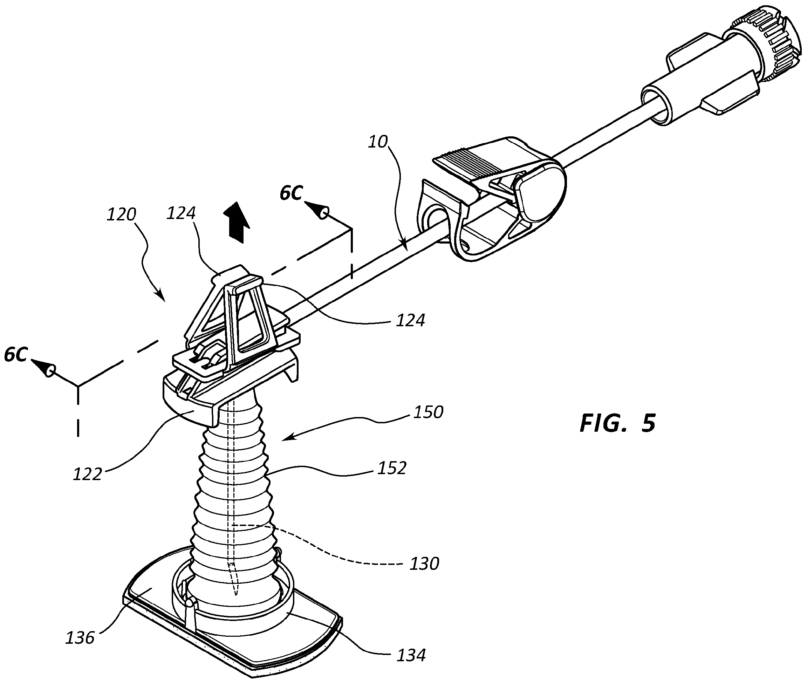

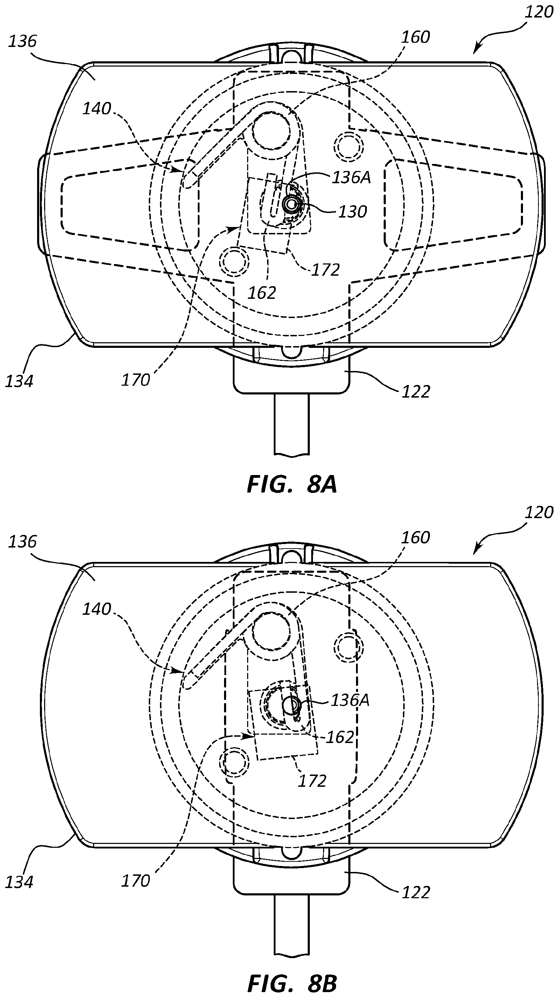

FIGS. 4 and 5 show the infusion set 10 including a needle assembly 120 according to another example, wherein the needle assembly includes a handle portion 122 with handles 124 extending therefrom. A needle 130 extends from the handle portion 122 and initially through a safety assembly 134 that is slidably disposed with respect to the needle 130 so as to be axially slidable therewith. The safety assembly 134 includes a base 136 that houses a needle safety component 140 (FIGS. 8A, 8B) for shielding a distal tip 130A of the needle 130 when use of the needle assembly is complete.

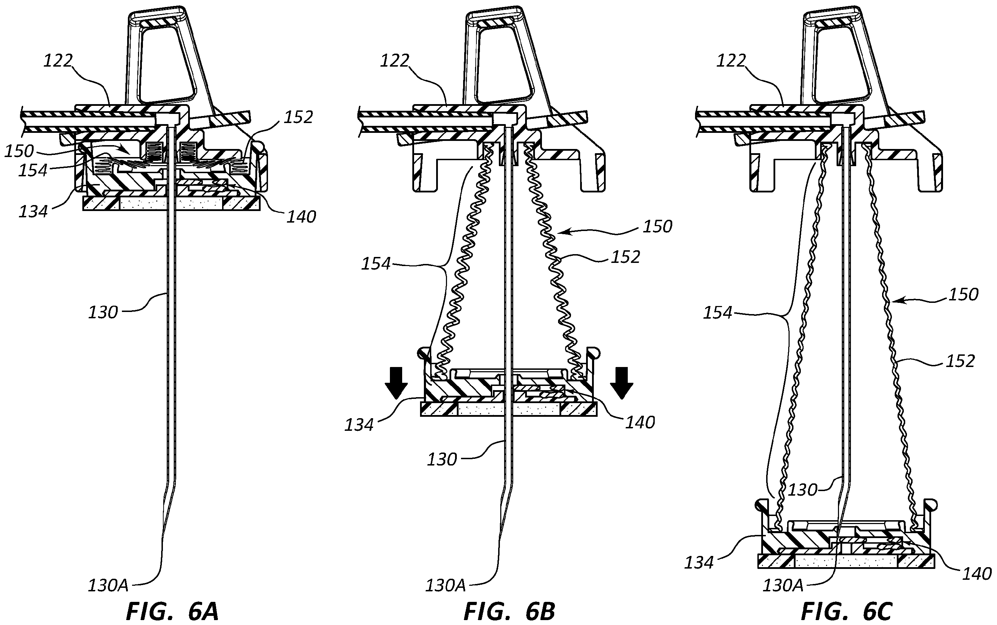

The needle assembly 120 further includes a fluid isolation component 150 for isolating any fluid or vapor that may unintentionally escape from the needle 130 during use of the needle assembly. Specifically, the fluid isolation component 150 in the present example includes a conically shaped, extensible shroud 152 disposed about the body of the needle 130 and extending between the handle portion 122 and the axially slidable safety assembly 134. Including plastic such as PET or other substantially impermeable, collapsible, and suitable durable material, the shroud 152 forms a hollow cone about the needle 130 and is corrugated with corrugations 154 in a bellows-like manner to enable it to fold up compactly when the safety assembly 134 is undeployed (FIG. 4) and to extend to cover and substantially encompass the needle 30 when the safety assembly 134 is deployed (FIG. 5)*, i.e.,* the safety assembly is axially slid down the needle 130 toward the distal tip 130A such that the needle safety component 140 shields the distal tip. FIGS. 6A-6C depict the manner of deployment of the safety assembly 134 and the extension of the corrugated shroud 152. In the extended state shown in FIGS. 5 and 6C, the shroud 152 assists in isolating fluids/vapors present on the needle 130 or emitted from the needle distal tip 130A from contact with the clinician.

Note that examples of safety needles that can utilize principles discussed here and in other embodiments herein can be found in the following United States patent and patent applications: Patent No. 7,717,888; Publication No. 2003/0114797; Publication No. 2008/0262434; and Publication No. 2010/0152677.

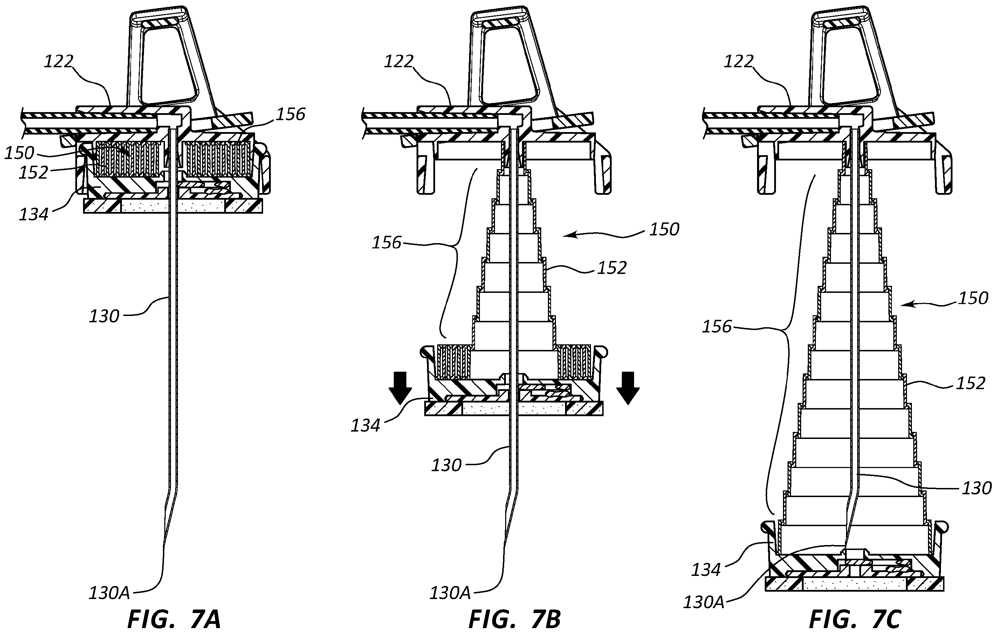

The shroud 152 as the fluid isolation component 150 can include other configurations. One such configuration is shown in FIGS. 7A-7C, wherein the shroud includes a plurality of interlocked, telescoping segments that are extendible to cover and encompass the needle body when the safety assembly 134 is deployed (FIG. 7C). When the safety assembly 134 is undeployed, the telescoping segments 156 are stacked together, as shown in FIG. 7A. Again, these and other configurations for encompassing the needle body illustrate manners by which a fluid isolation component can isolate the needle body and tip in order to prevent fluid exposure to clinician.

FIGS. 8A and 8B depict details of the needle safety component 140 of the needle assembly 120 of FIGS. 4-7C. Particularly, FIGS. 8A and 8B depict bottom views of the needle assembly 120. The needle safety component 140 is shown, including a coiled wire torsion spring 160 included within the base 136 of the safety assembly 134. The spring includes at one end thereof an obstruction component, *i.e.,* a looped portion 162 that is biased to lie against the needle 130 when the needle extends through a hole 136A defined in the base 136 of the safety assembly 134, as shown in FIG. 8A. As shown in FIG. 8B, once the distal tip of the needle 130 is withdrawn into the base 136 in connection with extension of the safety assembly *134 (e.g.,* FIGS. 5, 6C, 7C), the spring 160 expands such that the looped portion 162 slides over the needle hole 136A to prevent re-emergence of the needle distal tip.

In addition, a fluid isolation component 170 is included with the spring 160 for isolating any fluid or vapor that may unintentionally escape from the needle 130 during use of the needle assembly. Specifically, the fluid isolation component 170 includes a shield 172, shown in FIGS. 8A and 8B, which is attached proximate the looped portion 162 of the spring 160. Thus, when the looped portion 162 slides over to prevent re-emergence of the distal tip 130A of the needle 130 through the hole 136A (FIG. 8B), the shield fully covers and occludes the hole so as to prevent any fluid/vapor leaking from the distal tip of the needle from exiting through the hole and potentially contaminating the environment or clinician. The shield 172 thus serves to occlude the hole 136A and isolate any fluids/vapors from the clinician. Note that the particular size, shape, and configuration of the shield can vary from what is shown and described herein, as can the particular configuration of the needle assembly. In one embodiment, it is appreciated that the shield can include an absorbent material so as to absorb any leaked fluid.

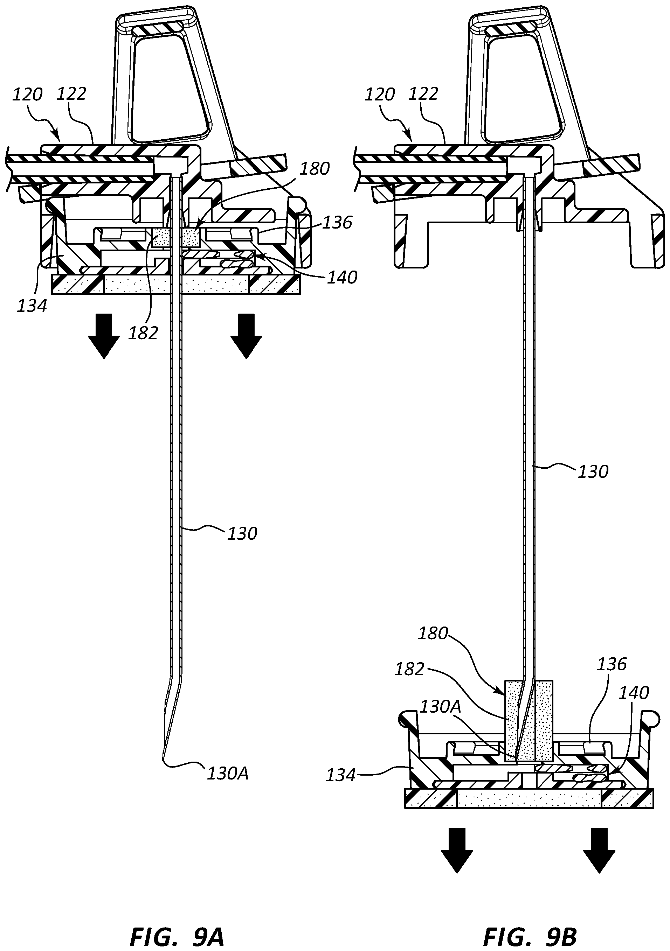

FIGS. 9A and 9B depict details of the needle assembly 120 according to another example, including a fluid isolation component 180 for isolating any fluid or vapor that may unintentionally escape from the needle 130 during use of the needle assembly. As shown, the fluid isolation component 180 in the present embodiment includes a cylindrical absorption plug 182 included with the axially slidable safety assembly 134 of the needle assembly 120 and including a central cavity so as to be positioned about a portion of the body of the needle 130 (FIG. 9A). The central cavity of the plug 182 is sized such that the plug is able to wipe the outer surface of the body of the needle 130 as the safety assembly 134 is axially slid down the needle toward the distal tip 130A thereof, thus removing fluid from the outer needle surface and capturing it in the plug itself. In addition, once the safety assembly 134 has fully shielded the needle distal tip 130A (FIG. 9B), the plug 182 is positioned about the distal opening of the lumen of the needle 130 so as to catch and absorb any fluids/vapors emanating therefrom.

It is appreciated that the absorption plug can include a variety of size, type, and material configurations, and can be employed on a variety of needle-based devices where residual fluid/vapor capture is desired. In one embodiment, for instance, the absorption member includes activated charcoal. In other embodiments, other materials and membranes can be employed, including silica gel, clays, activated alumina, zeolites, 0.2 micron or other filtration material, etc. The description included herein is therefore not intended to limit the present disclosure in any way.

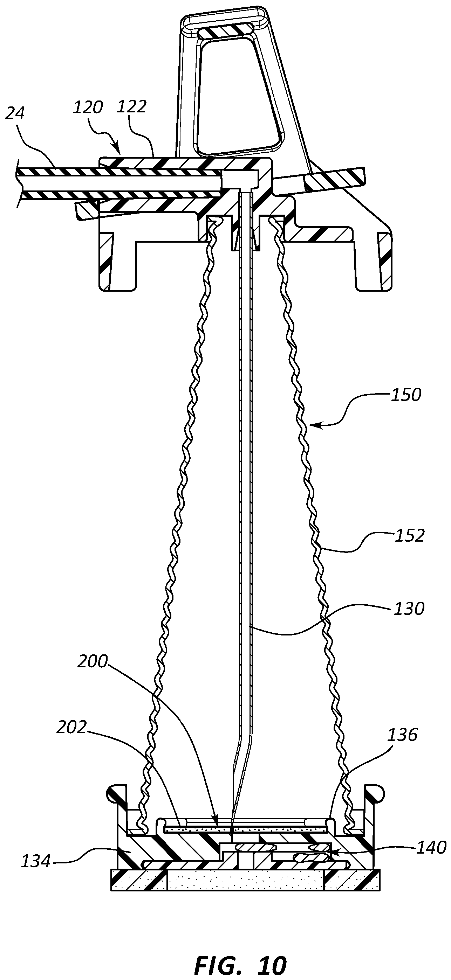

FIG. 10 shows details of a fluid isolation component 200 according to another example, including an absorption disk 202 included with the safety assembly 134. The absorption disk 202 is disposed above the needle safety component 140 in the safety assembly base 136 and is slit to enable the needle 130 to pass therethrough. Extension of the safety assembly 134 down the length of the needle 130 enables the absorption disk 202 to wipe the outer needle surface so as to remove any fluid present thereon. In addition, once the safety assembly 134 is fully extended to shield the needle 130 (FIG. 10), the absorption disk 202 is positioned so as to absorb any fluid/vapor leaking from the distal lumen opening at the needle distal tip 130A. As with the previous example, the absorption disk 202 in one example includes activated charcoal or other suitable, absorbent material as outlined above in connection with the absorption plug 182 shown in FIGS. 9A and 9B. The position, shape, thickness or other configuration of the absorption disk can vary from what is shown and described herein.

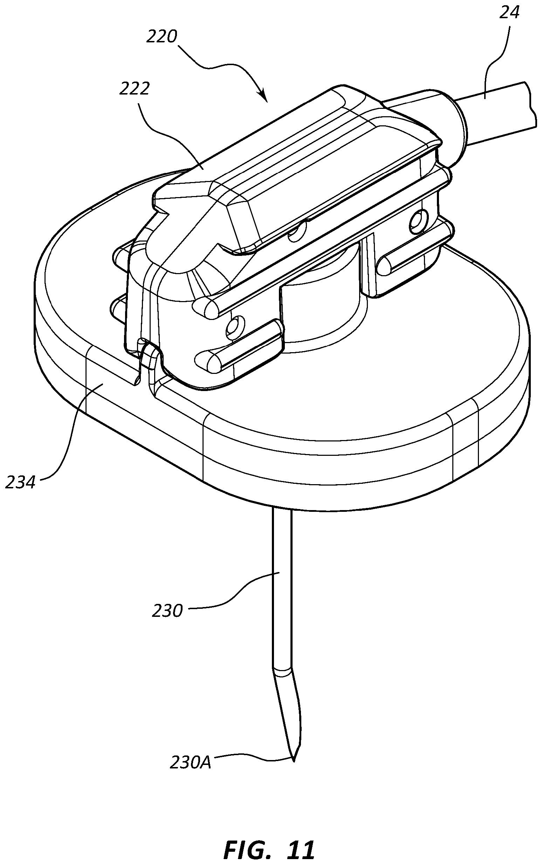

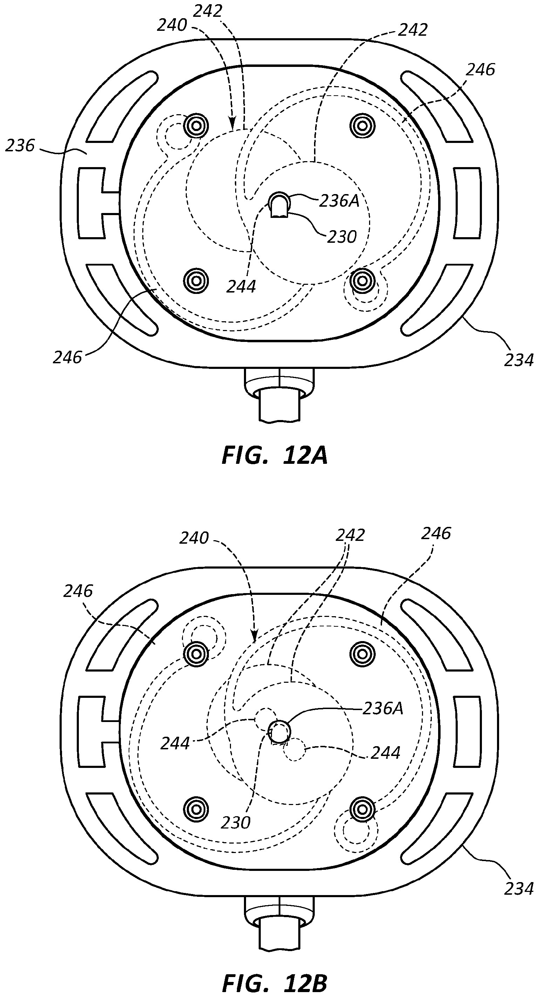

FIGS. 11-12B depict various details of a needle assembly 220 that can include a fluid isolation component, according to one example. As shown, the needle assembly 220 includes a handle portion 222 from which extends a needle 230. The needle 230 initially extends through a safety assembly 234 that is slidably disposed with respect to the needle so as to be axially slidable therewith. The safety assembly 234 includes a base 236 that houses a needle safety component 240 (FIGS. 12A, 12B) for shielding a distal tip 230A of the needle 230 when use of the needle assembly is complete.

In greater detail, FIGS. 12A and 12B show that the needle safety component 234 includes two spring-based shutters 242 that each define a hole 244 through which the needle 230 passes when the needle extends through the safety assembly 234 and out a hole 236A defined in the base 236, such as in the configuration shown in FIG. 11. The shutters 242 each further include a spring arm 246. As seen in FIG. 12A, when the safety assembly 234 is undeployed (FIG. 11), the holes 244 of the shutters 242 are aligned so that the needle 230 passes therethrough. This constrains the shutters 242 and spring arms 246 into the configuration shown in FIG. 12A.

When the safety assembly 234 is actuated, however, it is slid down the length of the needle 230 so as to cause the needle distal tip 230A to recede from the hole 236A and the shutter holes 244 so as to be shielded within the safety assembly base 236. As shown in FIG. 12B, this causes the shutters 242 to no longer be constrained by the needle 230 and enables the shutter spring arms 246 to slide the shutters laterally within the base 236 so as to cover and occlude the hole 236A defined in the base, thus preventing reemergence of the needle distal tip 230A. Note that further information regarding this and other related needle safety assemblies can be found in U.S. Patent No. 6,585,704 to Luther et al., titled "Method of Retaining a Tip Protector on a Needle with a Curved Tip."

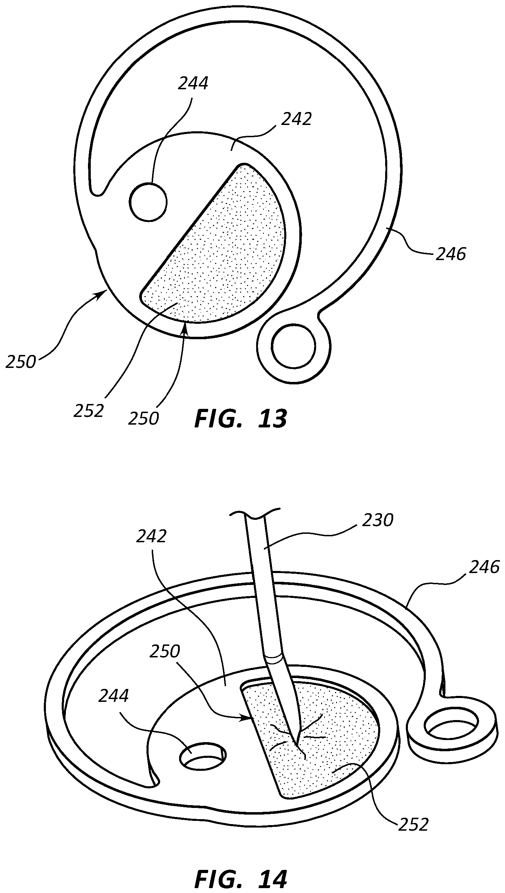

In accordance with one example the needle assembly 220 includes a fluid isolation component 250 for isolating any fluid or vapor that may unintentionally escape from the needle 130 during use of the needle assembly. Specifically, the fluid isolation component 250 in the present embodiment includes an absorption pad 252 disposed on a backside of one or both of the shutters 242 of the safety assembly 234. As shown in FIGS. 13 and 14, the pad 252 is disposed on the shutter 242 so that the distal tip 230A of the needle 230 rests against it after the distal tip has been withdrawn and shielded by the base 236 of the safety assembly 234. Should any fluid leak from the distal opening of the lumen of the needle 230, it can be readily captured by the pad 252, thus preventing its escape outside of the safety assembly 234. The pad can include one or more of suitable materials including those listed above in connection with the example of FIGS. 9A and 9B, silicone, rubber, etc. As shown, the pad can also be recessed within the shutter 242 so as to provide a basin for capture of the fluid, in one example. Note that the pad and shutters can vary in size, number, shape, design, etc.

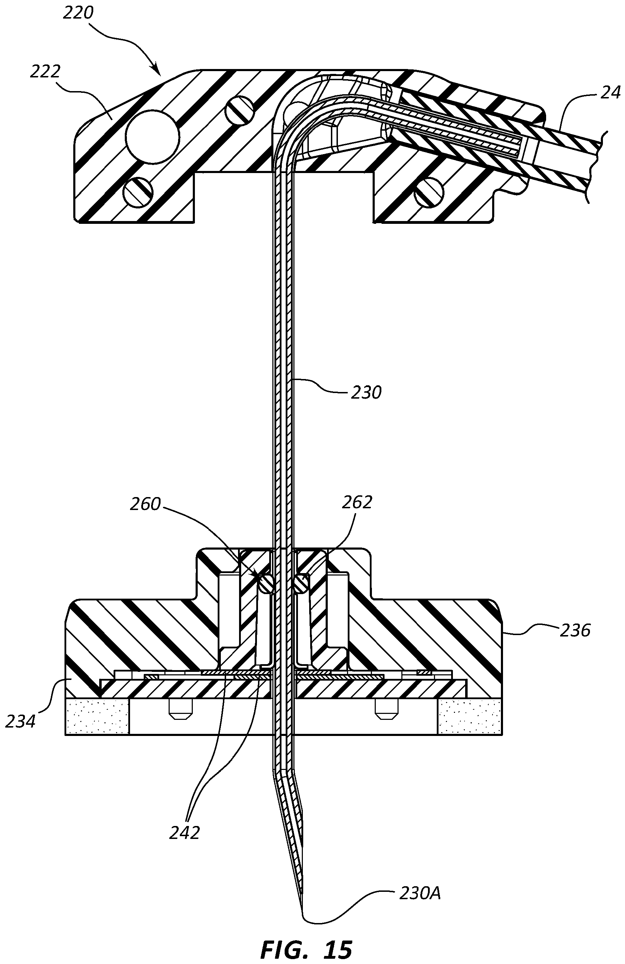

FIG. 15 shows the needle assembly 220 including a fluid isolation component 260 according to one example, wherein the fluid isolation component includes an O-ring 262 that is disposed within the safety assembly 234 about a portion of the needle 230. So positioned, the O-ring 262 wipes the length of the needle 230 when the safety assembly 234 is axially slid down the needle in order to shield the needle distal tip 230A. The O-ring 262 is sized such that its wiping action cleans the outer needle surface of any fluids that might otherwise be exposed to the clinician and prevents their escape from the safety assembly base 236. In one embodiment, the O-ring can be configured to be absorbent so as to soak up any fluid it comes into contact with during wiping of the needle. Note that the O-ring can be placed in other locations with respect to the needle safety assembly and that the needle housing and safety assembly can vary in configuration from what is shown.

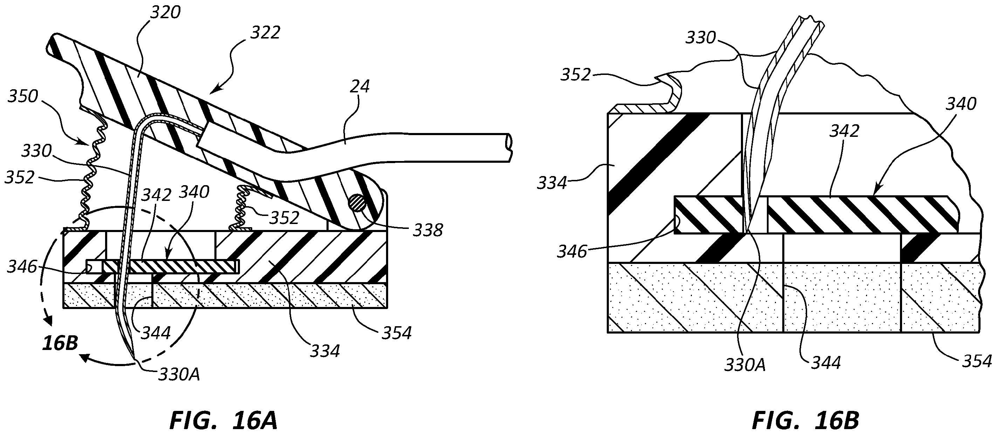

FIGS. 16A and 16B depict various details of a needle assembly 320 including a fluid isolation component, according to one example. The needle assembly 320 includes a handle portion 322 from which extends a needle 330. The needle 330 initially extends through a hole 344 defined in a safety assembly 334 that is pivotally movable with respect to the handle portion 322 and the needle 330 via a hinge point 338. The safety assembly 334 houses a needle safety component 340 including a laterally slidable shutter 342, disposed in a shutter cavity 346, for shielding a distal tip 330A of the needle 230 when use of the needle assembly is complete. A foam pad 354 is disposed on the bottom of the safety assembly 334.

As shown in FIG. 16B, the needle 330 is biased while residing in the hole 344 of the safety assembly 334 such that when the distal tip 330A is withdrawn from the hole, the needle 330 urges the shutter 342 to laterally slide within the shutter cavity 346, thus covering the hole and preventing re-emergence of the needle distal tip. In another example, the shutter itself can be biased to urge the needle distal tip laterally.

The needle assembly 320 further includes a fluid isolation component, here configured as an extensible shroud 352 that extends about the needle 330 between the handle portion 322 and the safety assembly 334 to isolate the body of the needle and any vapors present therewith. Thus, the shroud 352 provides isolation of fluids present on the needle 330. In addition, the shutter 342 provides some fluid isolation as well.

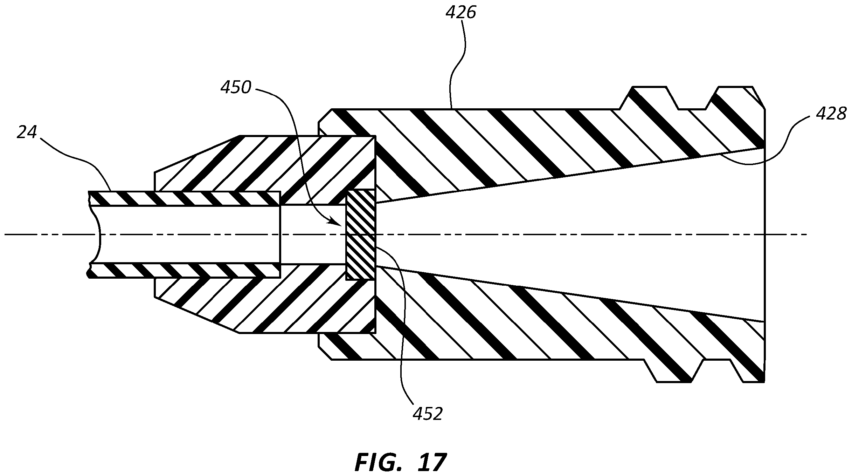

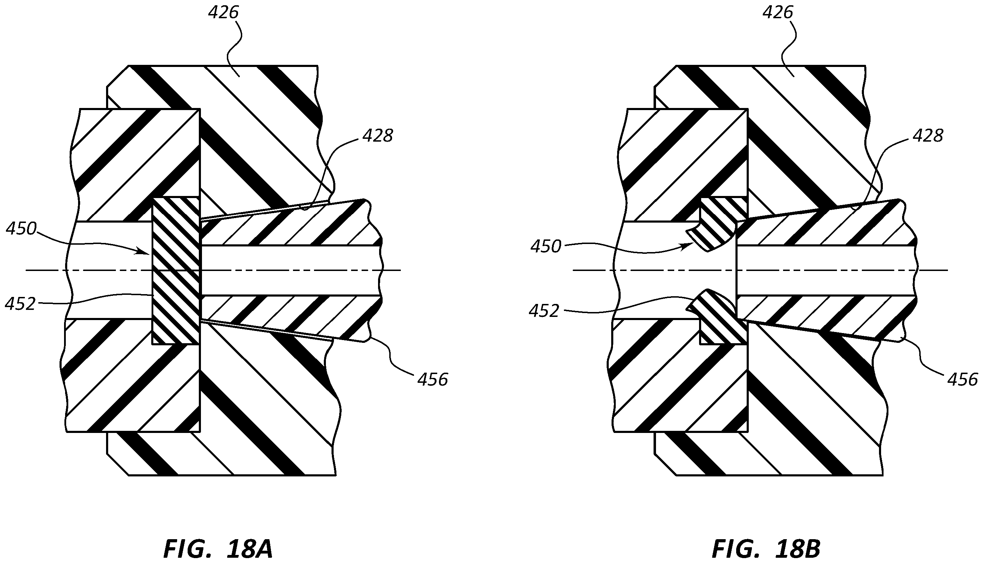

FIGS. 17-18B disclose a luer connector 426 including a fluid isolation component, according to one example. As shown, the connector 426 is a female-type luer connector, though the principles described here can be extended to other connective or fluid-carrying components of an infusion set or other suitable fluid delivery medical device. Connected to the extension leg tubing 24, the connector 426 includes a body that defines a cavity 428 suitable for receiving a male-type connector 456 (FIGS. 18A, 18B) therein. The connector 426 can include threads to enable the male connector 456 to threadably connect therewith. The cavity 428 defines a portion of a fluid pathway through the connector body.

A fluid isolation component 450 is included in the connector 426. In particular, the fluid isolation component 450 in the present embodiment includes a slit valve 452 that is disposed in the fluid pathway defined by the connector 426. Other suitable types of valves may also be employed.

As seen in FIGS. 18A and 18B, when the male connector 456 is received but not fully seated within the cavity 428 of the female connector 426, the valve 452 remains closed, thus isolating any fluid contained in the extension leg tubing 24 attached thereto. When the male connector 456 is fully inserted into the female connector 426, the distal end of the male connector engages and opens the valve 452, thus allowing fluid flow therethrough. This configuration of the connector 426 thus serves as one example a connector-based fluid isolation component; other configurations of this principle are contemplated.

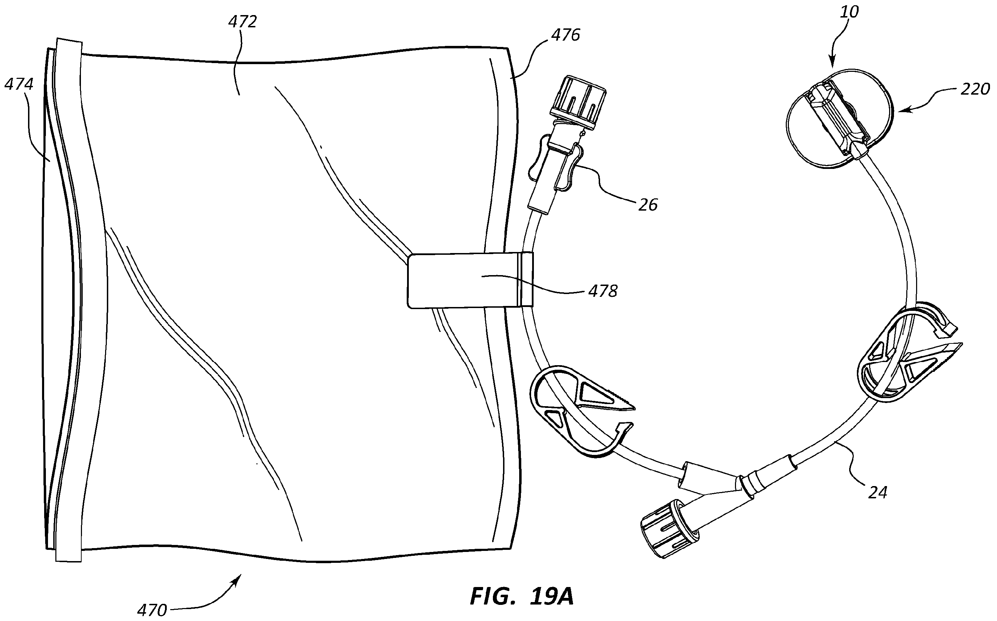

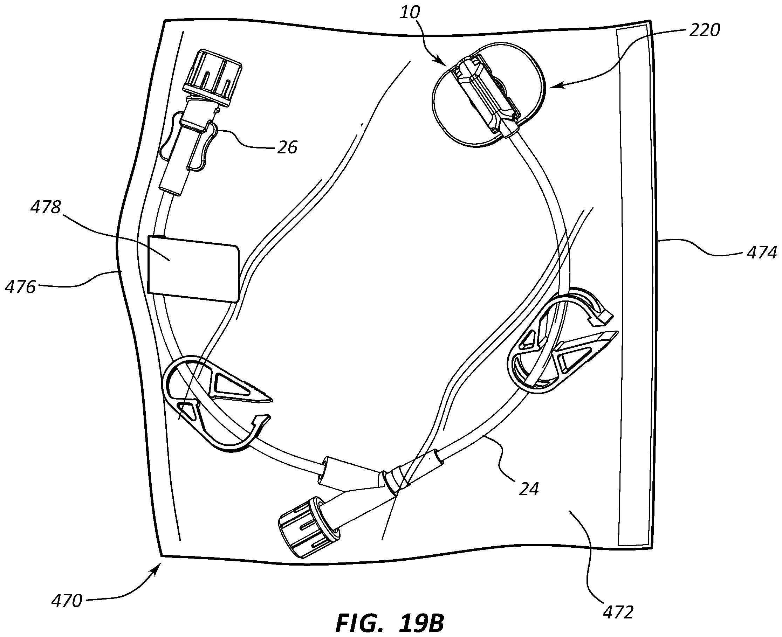

FIGS. 19A and 19B depict another example of a fluid isolation component for preventing unintended contact with fluid or vapors resulting from use of an infusion set. In particular, an infusion set 10 is shown, including a needle assembly 220, extension leg tubing 24, and luer connector 26. Also shown is a fluid isolation component 470, which in the present example includes a bag 472 of plastic or other substantially fluid-impermeable material. The bag includes a sealable open end 474 and a closed end 476. The bag 472 is attached to the tubing 24 of the infusion set 10 or other suitable component thereof via and adhesive strip 478 or other suitable connective apparatus.

The bag 472 is initially inside-out before use of the infusion set 10. Once use of the infusion set 10 has ended, the user reaches a hand through the open end 474 of the bag 472 and pulls the infusion set into the bag, turning the bag right side-out in the process. Once the infusion set 10 is fully within the bag 472, the open end 474 of the bag 472 is sealed, as seen in FIG. 19B, thus isolating the user from any fluids or vapors included on the needle assembly 220 or any other portion of the infusion set 10. Note that the bag can be configured in one or more sizes and shapes, can include one-time, resealable, or other suitable type of sealing mechanism, and can be included with the infusion set in a variety of ways, both attached and detached thereto. The bag in the present embodiment is transparent, though in other embodiments it need not be.

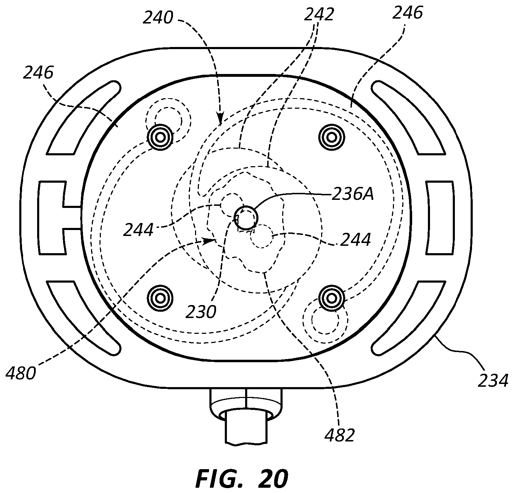

FIG. 20 depicts details of another possible fluid isolation component for use with the needle assembly 220 (shown in FIGS. 11-12B), or another suitable needle assembly. In particular, a fluid isolation component 480 is disclosed, including an amount of suitable viscous oil 482, such as silicone oil, interposed as a film between the shutters 242. When the needle 230 is retracted from the hole 236A in the needle assembly base 236, which retraction causes the shutters 242 to slide over and cover the hole, the oil 482 produces a fluid impermeable barrier layer between the shutters, thus preventing any fluid/vapor escaping the needle from escaping past the shutters. In other examples, other barriers can be employed between the shutters, including a gasket, O-ring, other compliant/elastomeric member, etc.

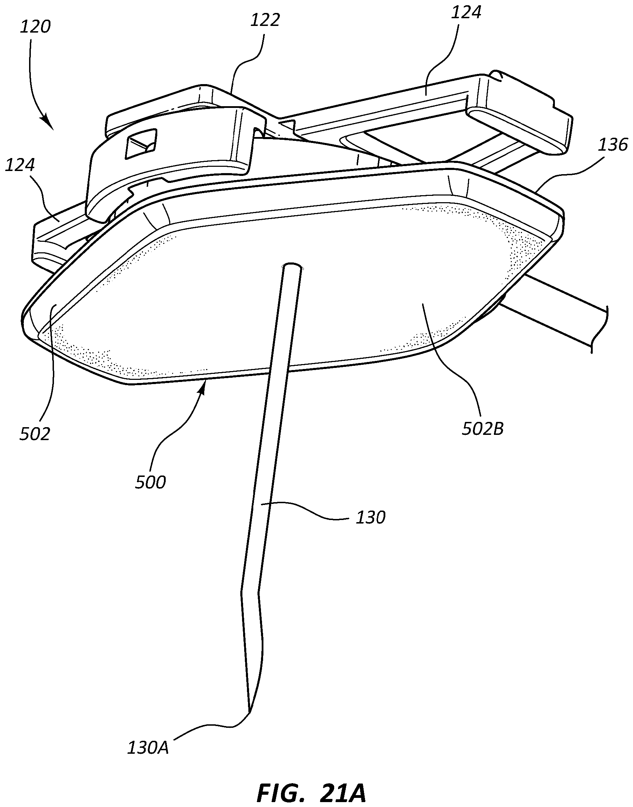

FIGS. 21A-24 depict various details regarding a fluid isolation component according to yet another example, for use with a safety needle assembly. As will be described, the fluid isolation component in the present example includes a self-sealing pad that prevents unintended leakage of fluids (e.g., liquids, gases) from the needle after use of the needle assembly. The self-sealing pad thus prevents undesired exposure to clinicians of potentially hazardous substances.

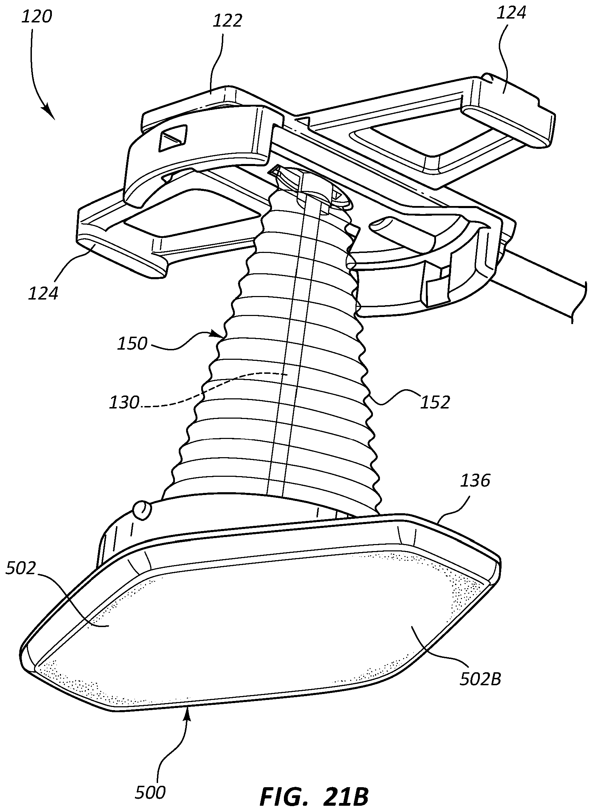

FIGS. 21A and 21B show the needle assembly 120 in similar configuration to that described further above in connection with FIGS. 5-6C. As before, the needle assembly 120 includes a first needle assembly portion, *i.e.,* the handle portion 122, with handles 124 extending therefrom. The hollow needle 130 extends from the handle portion 122 and initially through the safety assembly 134 that is slidably disposed with respect to the needle 130 so as to be axially slidable therewith. The safety assembly 134 includes the base, also referred to herein as a second needle assembly portion or the base portion 136, which houses the needle safety component 140 (FIGS. 8A, 8B) for shielding the distal tip 130A of the needle 130 when use of the needle assembly is complete.

As already described further above, the needle assembly 120 further includes a first fluid isolation component 150 for isolating any fluid or vapor that may unintentionally escape from the needle 130 during use of the needle assembly. Specifically, the first fluid isolation component 150 includes the conically shaped, extensible shroud 152 disposed about the body of the needle 130 and extending between the handle portion 122 and the axially slidable safety assembly 134 of the base portion 136. The shroud 152 forms a hollow cone about the needle 130 and is corrugated with corrugations 154 in a bellows-like manner to enable it to fold up compactly when the safety assembly 134 is undeployed (FIG. 4) and to extend to cover and substantially encompass the needle 30 when the safety assembly 134 is deployed (FIG. 5). As already discussed. in the extended state shown in FIGS. 5 and 6C, the shroud 152 assists in isolating fluids/vapors present on the needle 130 or emitted from the needle distal tip 130A from contact with the clinician.

FIGS. 21A-21B further show a self-sealing pad 500 disposed on a bottom external surface of the base portion 136. The pad 500 includes a self-sealing material that enables the needle 130 to extend therethrough, as seen in FIGS. 21A (also referred to as the first needle position), but seals when the needle is retracted back through the pad via separation of the base portion 136 from the handle portion 122, as seen in FIG. 21B (also referred to as the second needle position). In particular, and as shown in FIG. 21B, the full extension of the base portion 136 from the handle portion 122 causes the distal tip 130A of the needle 130 to be drawn through the pad 500 such that the distal tip is shielded within the base portion, in particular, shielded by the needle safety component 140. Because of its self-sealing characteristics, the pad 500 substantially seals the hole through which the needle 130 was disposed during needle assembly use, thus preventing any fluid leakage from the distal opening of the needle 130 to escape the base portion 136, as desired. Note that, though shown and described herein in connection with the needle assembly 120, the self-sealing pad 500 can be included with needle assemblies and infusion sets of a variety of configurations in addition to those discussed herein.

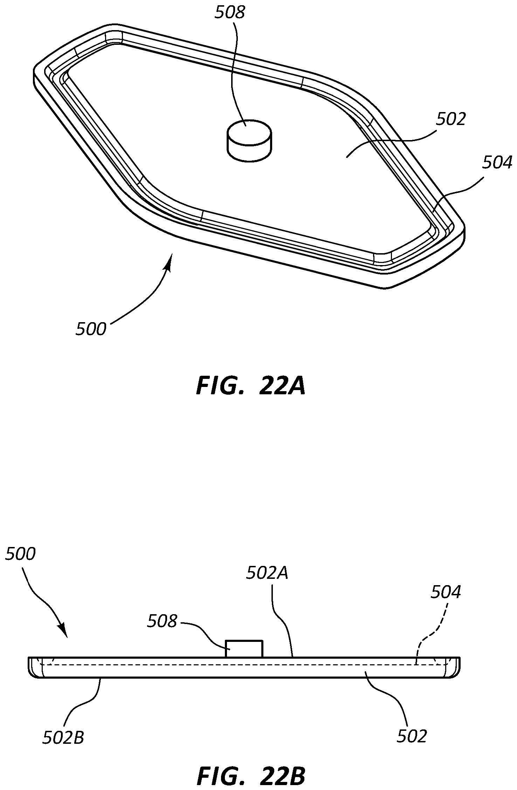

FIGS. 22A and 22B depict various features of the self-sealing pad 500 according to the present embodiment. As shown, the pad 500 includes a body 502 shaped to conform to the shape of the base 136, though in other embodiments the pad could include other shaped configurations. The body 502 defines an upper surface 502A that is configured to mate with the base portion 136 and a bottom surface 502B that serves as a skin-contacting surface for the needle assembly 120.

A groove 504 is defined about the perimeter and configured in the present embodiment to receive therein a corresponding protruded surface 506 defined on a bottom surface of the base portion 136. The receipt of the protruded surface 506 by the groove 504 assists in maintaining engagement of the pad 500 with the base portion 136, in one embodiment. The pad 500 can be affixed to the base portion 136 via a suitable adhesive or by other suitable methods, including mechanical fixation.

In one example, the pad 500 includes silicone, though other self-sealing materials, plastics, and elastomers can be employed. In one embodiment, a liquid silicone rubber ("LSR") that is injection molded then cured is employed to form the pad 500. So configured, the skin-contacting bottom surface 502B of the pad 500 provides a compliant surface to rest against the skin of the patient during use of the needle assembly 120.

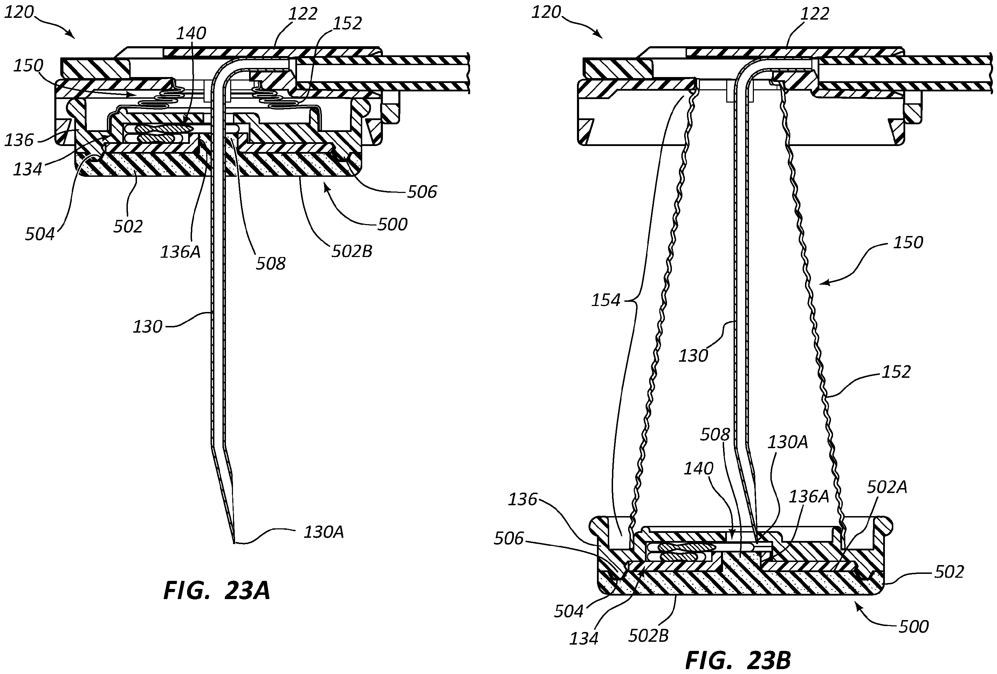

The pad body 502 further defines a centrally disposed raised portion 508, best seen in FIGS. 22A and 22B, which is shaped so as to compressively fit within an opening 136A that is defined in the base portion 136. As shown in FIGS. 23A and 23B, the raised portion 508 of the pad 500 is disposed within the opening 136A and is partially maintained in place via a compressive fit with the opening. So configured, the raised portion 508 acts as a septum to provide a robust fluid barrier when the needle 130 is withdrawn therethrough and shielded by the safety assembly 134, which occurs when the base portion 136 is selectively extended from the handle portion 122, as shown in FIG. 23B. Indeed, compression of the raised portion 508 by the opening 136A assists in the self-sealing characteristics of the pad 500 when the needle 130 is retracted, in one example. Thus, the self-sealing pad 500 serves as a second fluid isolation component, together with the shroud 152, for preventing fluid/vapor escape from the needle assembly. The shape and size of both the raised portion and the opening can vary from what is shown and described herein. In the present example, the distal tip 130A of the needle 130 fully withdraws from the raised portion 508, though in other examples the distal tip can remain in the raised portion 508 after shielding thereof by the safety assembly. An example of the latter configuration would include the distal tip of the needle retracting fully from the lower surface 502B of the pad body while still residing within raised portion 508 of the pad.

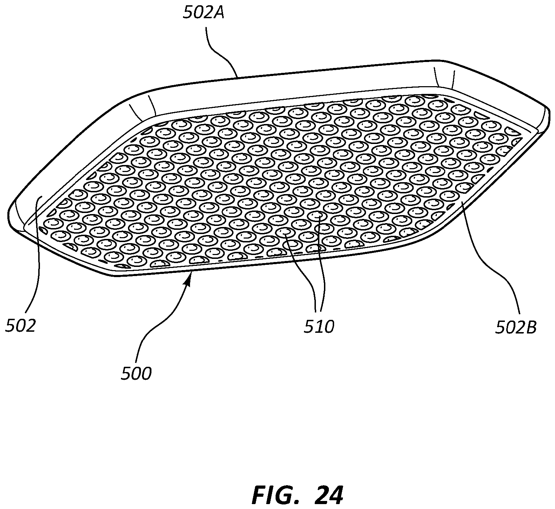

The self-sealing pad can be configured in other ways. For instance, FIG. 24 shows the bottom surface 502B as including a plurality of texture features 510 for increasing patient comfort while the needle assembly 120 is disposed on the skin of the patient. Also, in one embodiment, it is appreciated that the self-sealing pad can be treated so as to offer antimicrobial/antibacterial properties. Further, in one example, the bottom surface of the self-sealing pad can include an adhesive material to enable the pad to act as a securement device in maintaining the infusion set in place at a desired position on the patient's skin during use of the needle assembly. It is further appreciated that the needle assembly and accompanying infusion set can include one of many possible shapes, sizes, and configurations in addition to those shown and discussed herein.

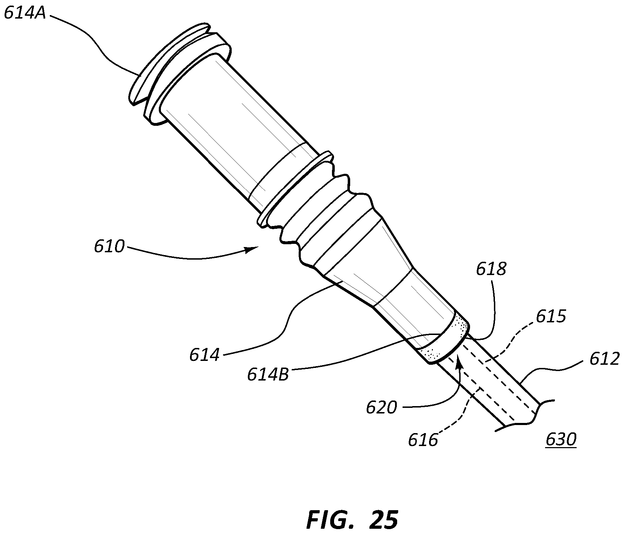

FIGS. 25-41 depict various features of an interface pad or other structure for inclusion on a medical device, such as a needle assembly or catheter assembly, so as to provide an interface between the medical device and the skin surface of the patient after the device has been percutaneously inserted into the patient via an insertion site. Particularly, the interface pad is positioned on the medical device so as to rest against the insertion site on the patient's skin once the device has been inserted into the patient.

Further, the interface pad includes one or more components that provide desirable effects at the insertion site. In accordance with one example, for instance, an antimicrobial agent and/or haemostatic agent are impregnated into the interface pad so as to provide antimicrobial and/or haemostatic properties to the insertion site via contact of the agents from the pad with the insertion site. In this way, infection, undesired bleeding, etc. can be controlled via use of the interface pad. In addition to the above-mentioned agents, other substances can be included in the interface pad to impart other desirable characteristics, such as antithrombogenic agents, for instance.

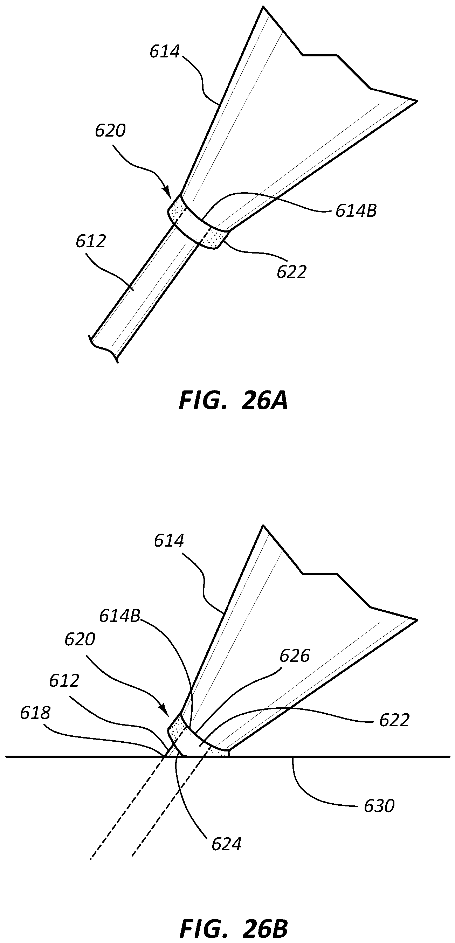

Reference is first made to FIG. 25, which shows a catheter assembly ("catheter"), generally designated at 610, according to one example. As shown, the catheter includes a hub 614 to which is connected at a distal end thereof an elongate, hollow catheter tube 612 defining a lumen 615. The catheter 610 includes a needle 616 inserted through the hub 614 so as to extend into the lumen 615 of the catheter tube 612. The needle 616 is used to assist with percutaneous insertion of the catheter tube 612 into the body of the patient via an insertion site 618 (FIG. 26B). Once the catheter 10 is percutaneously inserted within the patient, the hub 614 rests above a skin surface 630 of the patient, as seen in FIG. 26B. Note that, though shown here as defining a single lumen 615, the catheter tube 612 can define more than one lumen, such as two, three, or more lumens, in one embodiment. Also note that, though describing a catheter assembly herein, the present disclosure contemplates that other types of catheters including PICCs, PIVs, midline and intermediate dwell catheters, Foley and balloon catheters, epidural catheters, feeding catheters, drainage catheters, infusion sets, needle assemblies, and other medical devices can benefit from the teachings herein. The discussion to follow, therefore, is not intended to be limiting of the present disclosure.

FIG. 1 further shows an interface pad ("pad") 620, configured according to one example, attached to catheter 610. As shown, the pad 620 is interposed between the distal end 614B of the hub 614 and the skin surface 630 so as to rest adjacent to and substantially cover the insertion site 618 (FIG. 26B) when the catheter 610 is percutaneously inserted into the patient.

FIGS. 26A and 26B depict further details of the pad 620, according to one example. As shown in FIG. 26A, the pad 620 includes a compliant body 622 that can be permanently or temporarily attached to the distal end 614B of the hub 14, though other attachment locations to the medical device are possible. As will be seen, the pad body 622 can be configured in any one of a variety of shapes, sizes, etc. In the present example, the pad body 622 defines a disk-shaped configuration and is adhered by an adhering surface 626 to the hub distal end 614B via a biocompatible adhesive, though other modes of attachment, including chemical, mechanical, frictional, etc., can be employed. Example adhesives in one example include cyanoacrylate, UV- or heat-cured adhesives, epoxies, solvent bonding adhesives, acrylic-based, silicone-based, rubber-based, urethane-based, and hydrocolloid adhesives. In other examples, the interface pad is not compliant or compressible, but substantially rigid. For instance, an incompressible polymeric material that includes the ability to leach one or more agents (discussed below) for treating the insertion site 618 could be employed for the pad body 622. These and other variations of the pad are therefore contemplated.

Though a variety of suitable, biocompatible materials can be employed, in the present example the pad body 622 includes a compressible polyurethane foam that is capable of absorbing and holding agents (discussed below) that may be impregnated into the foam. Manufacture of the pad body 622 from a compressible/compliant material enables the pad to conform to the skin surface 630 about the insertion site 618 when the catheter tube 612 is percutaneously inserted into the patient's body. As such, one or more portions of an outer surface of the pad body 622 serve as a deformable contact surface 624 so as to provide a cushioning interface between the catheter hub 614 and the patient skin 630, as seen in FIGS. 25 and 26B. The cushioning effect of the pad body 622 serves to increase patient comfort when the catheter is disposed in the patient.

Specifically, in one example, the pad body 622 includes an aromatic polyether polyurethane foam, including a TDI or MDI hard segment and a PTMEG (polytetramethylene ether glycol) soft segment. Other suitable polymer-based foam materials as well as non-foam materials can also be employed for the pad body 622. In one example, desired characteristics for a foam material used for the pad body 622 include hydrophilicity (absorptive), a suitably large surface area to volume ratio for the foam, and a suitable diffusion coefficient. These desired characteristics are useful when one or more agents for treating the insertion site 618 are included with the pad body 622, as will be described below. In addition, other materials can be employed, including polyethylene, woven and non-woven fabrics including felt and cotton, gels, and hydrogels. In one example, the pad body includes a compressed foam that expands in size upon activation with blood or other body fluid/liquid. In such an example a dry antimicrobial or other agent can be included with the pad body.

As mentioned above, the pad 620 is configured in one example to include one or more agents for treating the insertion site 618 of the catheter 610 or other medical device. In one example, a liquid solution (or other suitable medium) including an antimicrobial agent, a haemostatic agent, an antithrombogenic agent, or other substance to protect, heal, or otherwise assist care of the insertion site 618 is included in the pad body 622. In the present example, the pad 620 includes a liquid antimicrobial agent that is infused during manufacture into the polyurethane foam material from which the pad body 622 is formed. Once the catheter 610 has been placed percutaneously into the patient and the pad 620 is positioned adjacent the patient skin 630 as shown in FIG. 26B, the antimicrobial agent previously infused into the foam pad body 622 contacts the insertion site 618 via the contact surface 624 of the pad body 622 and provides antimicrobial effect, thus desirably protecting the insertion site from infection.

In the present example, a liquid haemostatic agent is also infused during manufacture into the polyurethane foam material from which the pad body 622 is formed. When the pad 620 is positioned adjacent to the insertion site 618 as just described and as shown in FIG. 26B, the haemostatic agent previously infused into the foam pad body 622 contacts the insertion site via the contact surface 624 of the pad body 622 and provides haemostatic effect, thus desirably preventing excessive bleeding from the insertion site. Note that the pad body 622 in one example is absorptive so as to take up effusion of blood and other fluids from the insertion site 618, in one embodiment.

In one example, the antimicrobial agent can include silver, copper (and other biocompatible antimicrobial metals, silver sulfadiazine, chlorhexidine, chlorhexidine gluconate ("CHG"), chlorhexidine acetate ("CHA"), other suitable chlorhexidine-based antimicrobial agents, isopropyl alcohol ("IPA"), etc. In one example, the haemostatic agent includes microdispersed oxidized cellulose, other suitable hydrocolloids, etc. In one example embodiment, a solution included in the pad 620 includes an antimicrobial agent of about 11% by weight CHG and a haemostatic agent of about 8% by weight microdispersed oxidized cellulose, though these percentages can vary in other formulations. For example, in one example, the amount of CHG by weight in the solution can vary between about 11% to about 25%, though other ranges are possible, depending on various factors, including the type of pad material employed, processing parameters during pad manufacture, etc. In another example, it is appreciated that the antimicrobial agent, haemostatic agent, or other agent included with the interface pad can be in a dry state, such as a solid or powder, for instance.

In yet another example, the foam itself of the interface pad body can be configured to impart haemostatic properties and therefore act as the haemostatic agent. Indeed, in one example, negatively charged sulfonate groups can be incorporated into the soft segment (e.g., polytetramethylene ether glycol) of a polyurethane foam so as to impart haemostatic properties thereto. In such a case, no other haemostatic agent need be added, though an additional agent could be, if desired.

Further details regarding antimicrobial and haemostatic agents that may be used according to one embodiment are found in U. S. Patent Application Publication No. 2013/0110025, filed July 4, 2011, and titled "Dressing Device for Use with a Cannula or a Catheter,". In yet another example, the antimicrobial agent (such as CHG) is incorporated into the material from which the interface pad body is composed, such as a solvent acrylic adhesive. Details regarding such a configuration can be found in U. S. Patent application Publication No. 2013/030656, filed January 23, 2012, and titled "Chlorhexidine Gluconate Containing Solvent Adhesive,". In yet another example, an antimicrobial silicone adhesive, such as that produced by Covalon Technologies Ltd., Mississauga, Ontario, can be used to form the interface pad body and antimicrobial agent. In yet another example, a hydrogel or other hydrocolloid in which the antimicrobial and/or haemostatic agent is incorporated can be used to form the interface pad body.

It is appreciated that one or more of a variety of agents can be included with the pad 620 to render to it desirable qualities or characteristics. For instance, the pad body can include an antithrombotic agent. Note also that in one embodiment, the antimicrobial/haemostatic agent can be incident on the insertion site 618 via liquid dispersion.

It is further appreciated that, in one example, the afore-mentioned desirable qualities of the pad body material - including hydrophilicity/absorptiveness and sufficiently large surface area to volume ratio - assist in enabling the microdispersed oxidized cellulose haemostatic agent to be present in the pad body and to contact and interact with the insertion site, as desired. Additionally, in one example the afore-mentioned desirable quality of suitable diffusion coefficient for the pad body material assists in enabling the antimicrobial agent to contact and interact with the insertion site so as to provide desired antimicrobial properties.

In addition to agent-based protection, the pad 620 also physically protects the insertion site 618 by providing a physical barrier and cushion for the insertion site, which provide patient comfort when the catheter 610 or other medical device is resting against the skin, as seen in FIG. 26B. As such, it is appreciated that the pad can be manufactured using one or more of a variety of materials and in a variety of shapes, sizes, and configurations. In one example, as mentioned, the pad body includes a gel or hydrogel material.

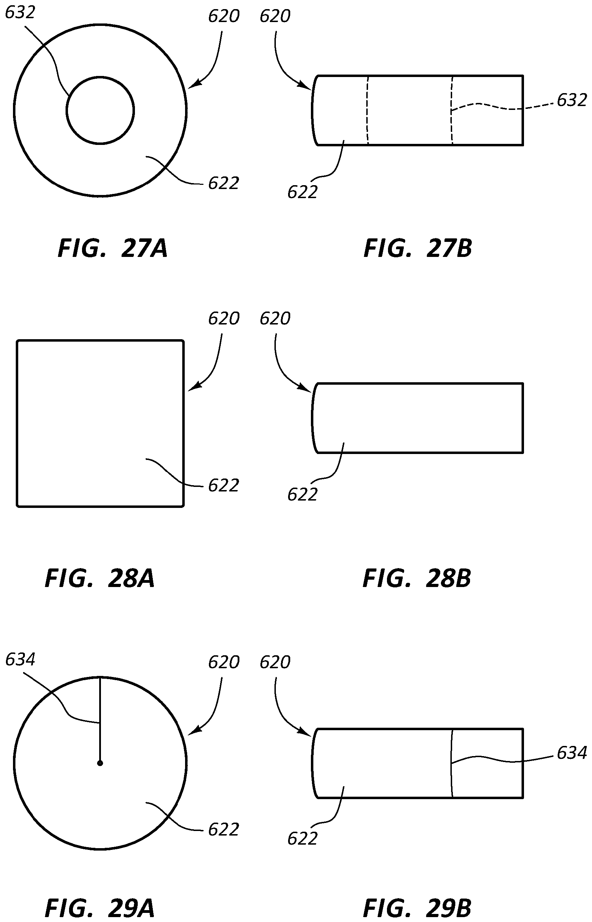

In light of the above, FIGS. 27A-29B depict various examples of possible shapes for the body 622 of the pad 620, according to examples. FIGS. 27A and 27B show the pad body 622 defining a central hole 632 for passage therethrough of the needle 616 (FIG. 25), similar to the pad configuration of FIGS. 25-26B. In one example, the thickness of the pad body 622 of FIGS. 27A and 27B is about 1.5875 mm (0.0625 inch), though other thicknesses are possible. Generally, the thickness of the pad body should be such that sufficient absorption of the antimicrobial and/or haemostatic agents is possible, while not becoming too thick so as to decrease the useful length of the catheter tube.

FIGS. 28A and 28B show a rectangular pad body 622, wherein a hole therethrough for passage of the catheter 610 may be made by piercing the pad body with the needle 616 during the catheter insertion procedure or during manufacture.

FIGS. 29A and 29B show a disk-like pad body 622 with a slit 634 defined from an edge of the disk to the center thereof to enable its attachment to or removal from the catheter 610. As such, it is appreciated that the pad 620 can be permanently or removably attached to the catheter hub 614, such as is seen in FIG. 26A or in another suitable location, as appreciated by one skilled in the art. These and other suitable pad body shapes and configurations are therefore contemplated.

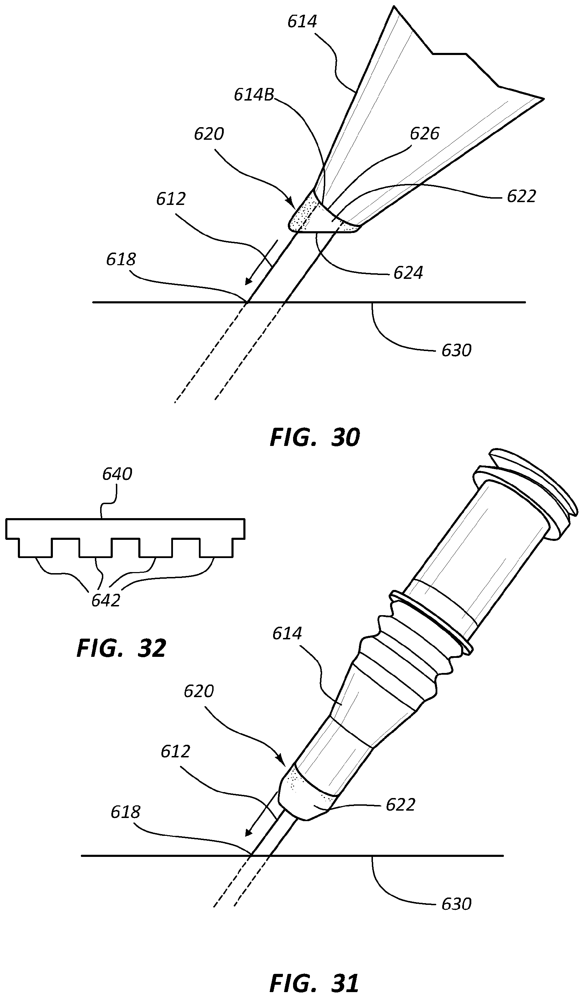

FIG. 30 depicts details of another possible shape configuration for the pad 620, wherein the pad body 622 defines an angular shape, from the perspective shown in FIG. 30, such that the contact surface 624 thereof is substantially parallel to the skin surface 30 when the catheter tube 12 is percutaneously inserted into the patient, as shown. Such a configuration distributes the contact load of the pad 620 with the patient skin 630 across the relatively parallel and flat contact surface 624, thus diminishing contact force at any given point on the contact surface, which increases patient comfort, and increases the relative surface area of the contact surface about the insertion site to enhance the effectiveness of the antimicrobial and/or haemostatic agents.

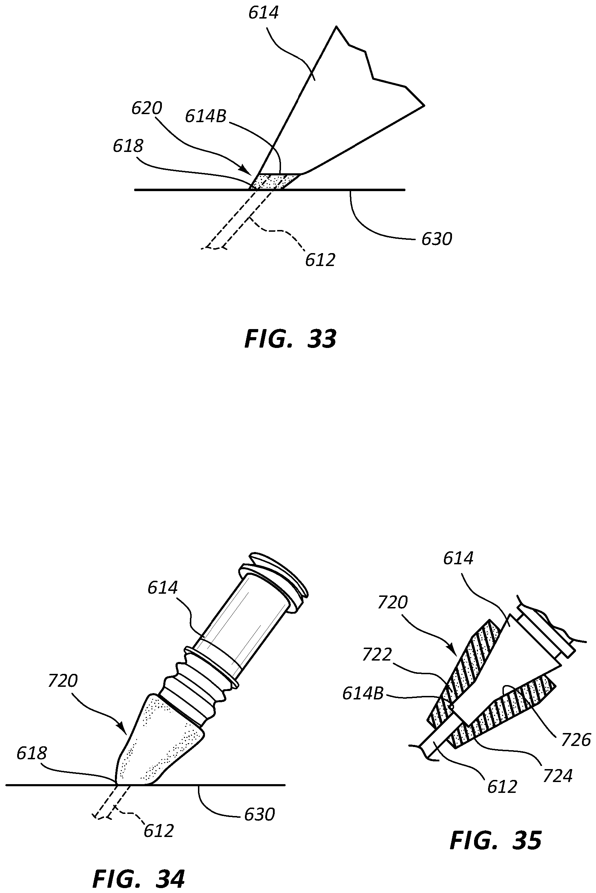

FIG. 33 depicts yet another possible pad configuration to maximize skin contact, wherein the pad body 622 approximately defines a parallelogram shape, from the perspective shown in FIG. 33, which also desirably produces a relatively parallel pad contact surface with respect to the skin surface 30. FIG. 33 further shows that, in one example, the distal end 614B of the hub 614 can be angled to be substantially parallel with the skin surface 630 after percutaneous catheter insertion is completed.

FIGS. 31 and 32 depict details of another example of the interface pad 620, wherein the pad is produced from a foam strip 640 that includes a pattern of spaced-apart teeth 642, as seen in FIG. 32. During pad manufacture, the foam strip 640 is rolled about the proximal end 612A of the catheter tube 612 and secured in its rolled configuration (via adhesive or the like) to define the interface pad 620 shown in FIG. 31. The toothed pattern of the foam strip 640 imparts an angled configuration for the pad body, which assists in providing a contact surface for the pad that is relatively more parallel to the skin surface 630 of the patient, as has been discussed. Note that the teeth 642 shown here is just one of a variety of different patterns that can be included in the foam strip 640 from which the pad 620 can be manufactured.

FIGS. 34 and 35 depict details of an interface pad 720 according to one example. As shown, the pad 720 includes a body 722 that defines a cavity 726 so as to form a cap-shaped configuration. So configured, the cavity 726 of the pad body 722 receives a portion of the catheter hub 614 therein such that the distal end of the hub is covered, or capped, by the pad. The pad 720 can be temporarily or permanently secured to the hub 614 along an adhering surface 726 via adhesive or other suitable fixation mode. The pad 720 defines an outer contact surface 724 for deformable contact with the skin surface 630. As before, the pad 720 can include an antimicrobial, haemostatic, or other suitable agent for protecting/healing the insertion site 618 of the catheter 610 and for destroying microbes that may otherwise be present on portions of the catheter tube 612 in contact with the interface pad. Also, in one example, the pad 720 can be scored or include a slit so as to make it readily removable from the catheter hub or other medical device to which it is at least temporarily attached.

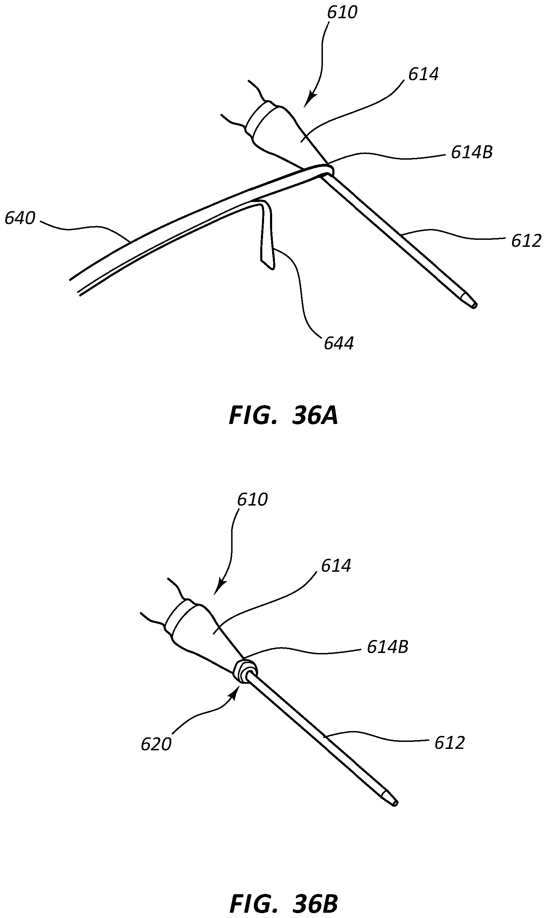

FIGS. 36A and 36B depict details of the interface pad 620 according to one example, wherein the pad is produced from a foam strip, such as the foam strip 640 shown in FIG. 36A. As shown in FIG. 36A, during pad manufacture, the foam strip 640 is rolled about the catheter tube 612. The foam strip 640 can include a liner 644 that is peeled away during rolling such that an adhesive under the liner adheres the pad to the catheter tube 612 and/or hub 614. Once the foam strip 640 has fully encircled the catheter tube 612/hub 614, it can be cut to define the pad 620 shown in FIG. 36B. These and other pad manufacturing methods are therefore contemplated. The foam strip 640 can include one of a variety of lengths, thicknesses, compositions, and other configurations.

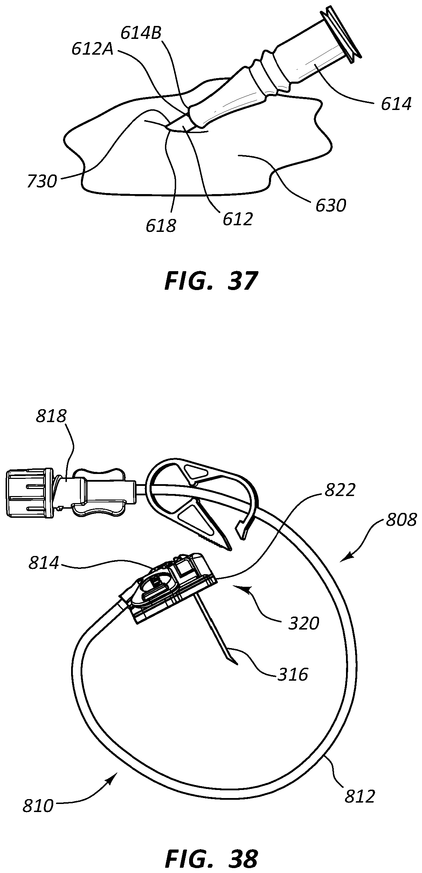

FIG. 37 shows yet another example for providing an antimicrobial/haemostatic interface for the catheter 610, wherein a proximal portion of the catheter tube 612 adjacent the proximal end 612A thereof is coated to provide a coated portion 730 that includes an antimicrobial, haemostatic, and/or other suitable agent for protecting and/or healing the insertion site of the catheter 610 through the skin 630 of the patient. The amount of the proximal portion of the catheter tube that is coated to form the coated portion 230 can vary according to need, catheter size, amount of the catheter to remain outside the patient body, etc., but in one example, the coated portion 730 extends distally from about one to about five millimeters from the distal end 614B of the hub 614.

FIGS. 38-39D depict various details of an interface pad for use with yet another type of percutaneous device, according to embodiments of the present invention. In particular, FIGS. 38-39D show a safety needle infusion set ("infusion set") 808, typically used to provide percutaneous access to a subcutaneously implanted access port. As shown, the infusion set 808 includes a needle assembly 810 from which extends tubing 812. The tubing 812 is operably connected with a needle hub 814 of the needle assembly 810 so as to be in fluid communication with a needle 816 that extends from the needle hub. The needle hub 814 includes an extensible base 815 that in turn defines a bottom surface 815A. The base is included as part of a needle safety component to prevent needle sticks to the user by the distal tip of the needle, in one embodiment. A connector 818 is included on the opposite end of the tubing 812. As mentioned, the needle 816 is configured to percutaneously pierce the patient's skin via an insertion site so as to access a subcutaneously implanted access port.

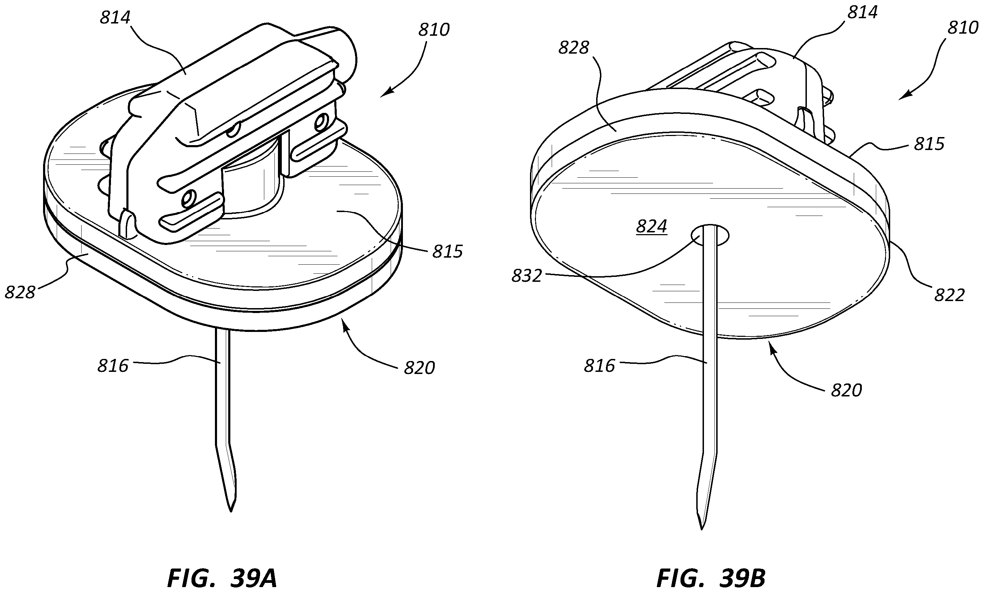

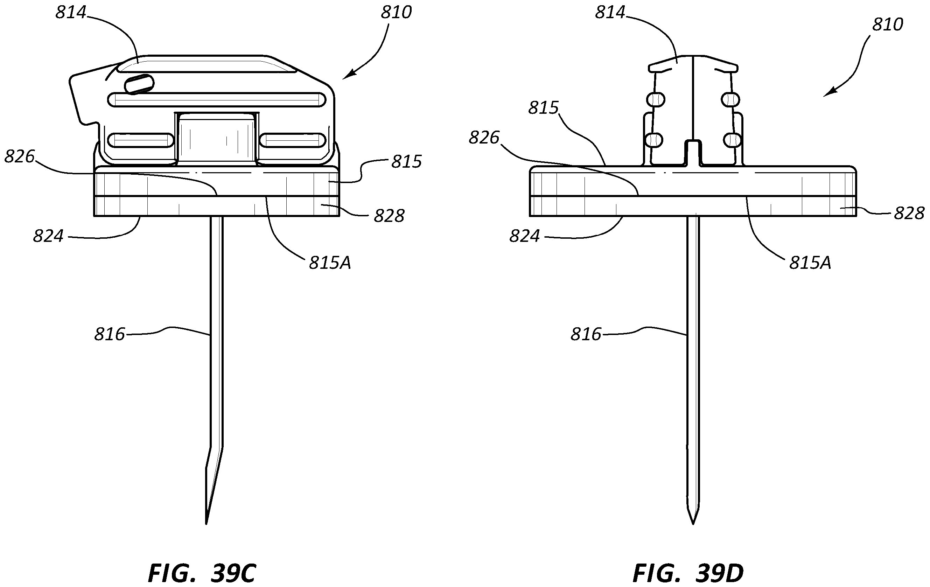

In accordance with the invention, the needle assembly 810 includes an interface pad 820 included on the bottom surface 815A of the needle hub base 815. As seen in FIGS. 39A-39D, the interface pad includes a body 822 and a hole 832 through which the needle 816 extends. The composition of the pad body 822 and the antimicrobial/haemostatic agents it contains is similar to what has been described in the examples above.

The body 822 of the interface pad 820 in the present embodiment defines a perimeter 828 that is shaped to correspond to the perimeter shape of the needle hub base 815. The interface pad body 822 further includes an adhering surface 826 where the pad body attaches to the bottom surface 815A of the needle hub base 815, and a contact surface 824. In the present embodiment, the adhering surface 826 of the pad body 822 is permanently attached to the bottom surface 815A of the needle hub base 815 via a suitable adhesive or other mode as discussed herein. In another embodiment, the pad body 822 is removably attached. The contact surface 824 rests against the skin when the infusion set needle 816 is percutaneously disposed within the patient. As has been described, this enables the antimicrobial/haemostatic agents included with the interface pad 820 to be in contact with and protect the needle insertion site.

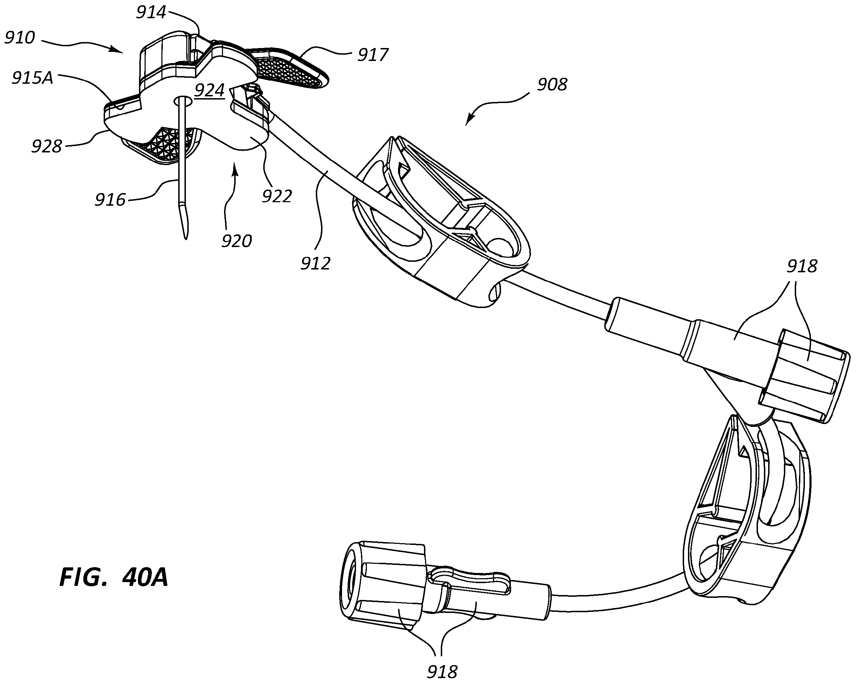

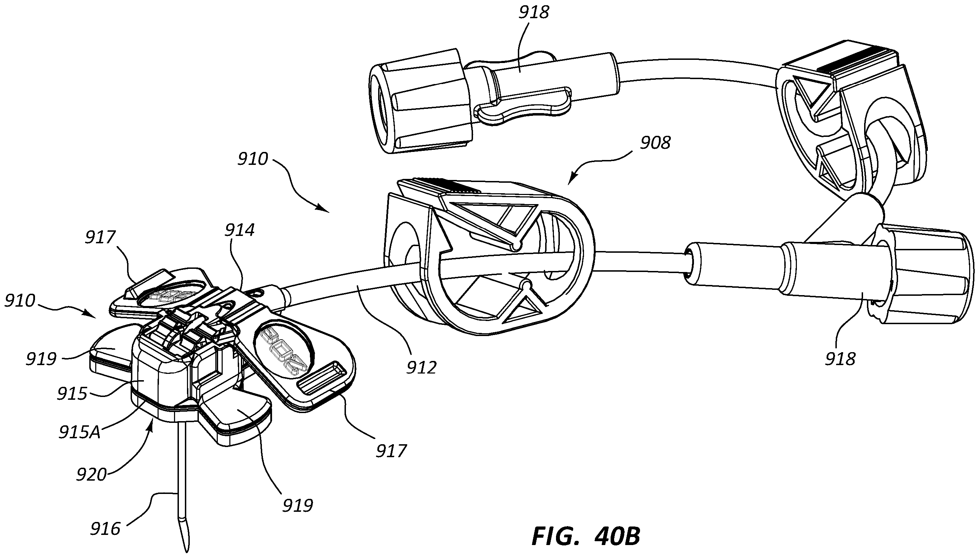

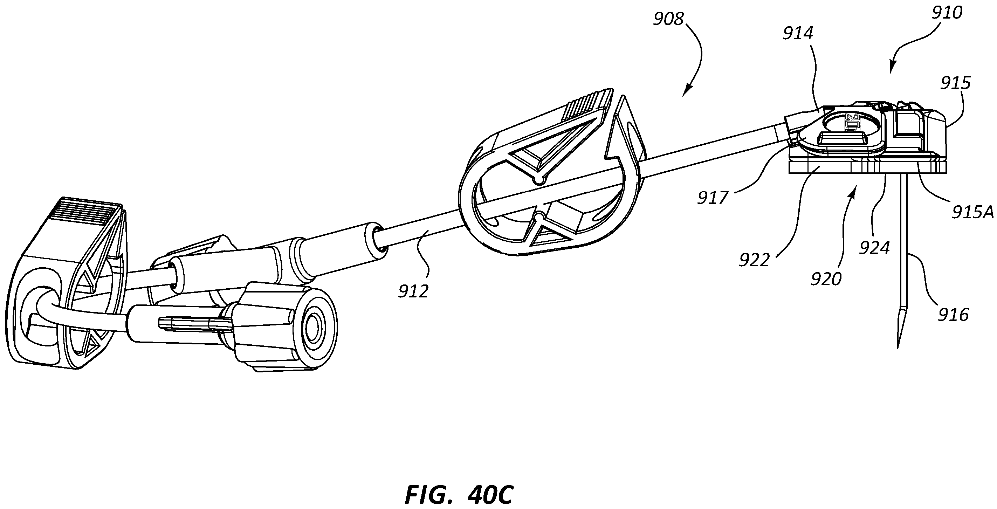

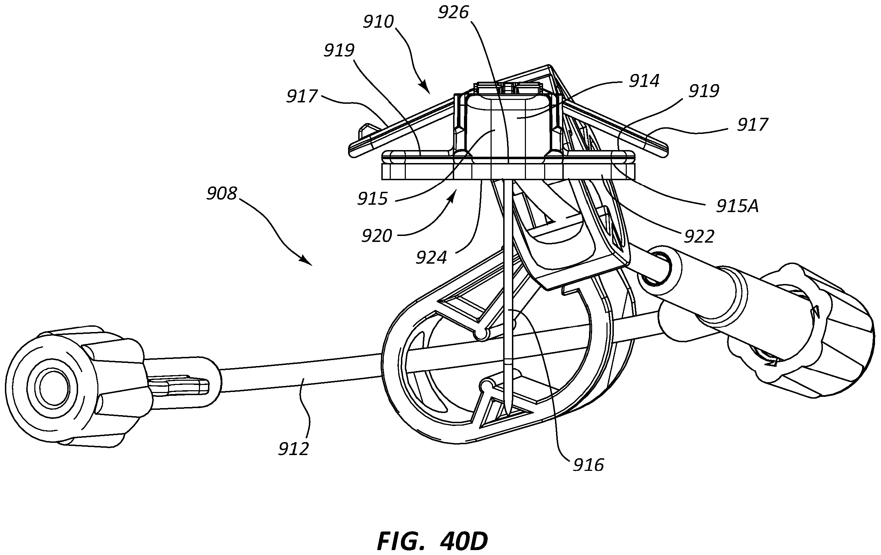

FIGS. 40A-40E shows an infusion set 908 and interface pad 920 according to another embodiment. As with the previous embodiment, a safety needle infusion set ("infusion set") 908 is shown and includes a needle assembly 910 from which extends tubing 912. The tubing 912 is operably connected with a needle hub 914 of the needle assembly 910 so as to be in fluid communication with a needle 916 that extends from the needle hub. The needle hub 914 includes an extensible base 915 that in turn defines a bottom surface 915A. The needle hub 914 further includes a pair of wings 917 for grasping the needle assembly 910. The base 915 includes a pair of extensions 919 to assist with using the infusion set 908. Note that the particular shape and configuration of the needle assembly can vary from what is shown and described herein. One or more connectors 918 are included on the opposite end of the tubing 912. As mentioned, the needle 916 is configured to percutaneously pierce the patient's skin via an insertion site so as to access a subcutaneously implanted access port.

In accordance with the invention, the needle assembly 910 includes an interface pad 920 included on the bottom surface 915A of the needle hub base 915. As seen in FIGS. 40A-40E, the interface pad includes a body 922 and a hole 932 through which the needle 916 extends. The composition of the pad body 822 and the antimicrobial/haemostatic agents it contains is similar to what has been described in the examples above.

The body 922 of the interface pad 920 in the present invention defines a perimeter 928 that is shaped to correspond to the perimeter shape of the needle hub base 915, including the shape of the extensions 919 of the needle hub base. The interface pad body 922 further includes an adhering surface 926 where the pad body attaches to the bottom surface 915A of the needle hub base 915, and a contact surface 924. In the present invention, the adhering surface 926 of the pad body 922 is permanently attached to the bottom surface 915A of the needle hub base 915 via a suitable adhesive or other mode as discussed herein. In another example, the pad body 922 is removably attached.

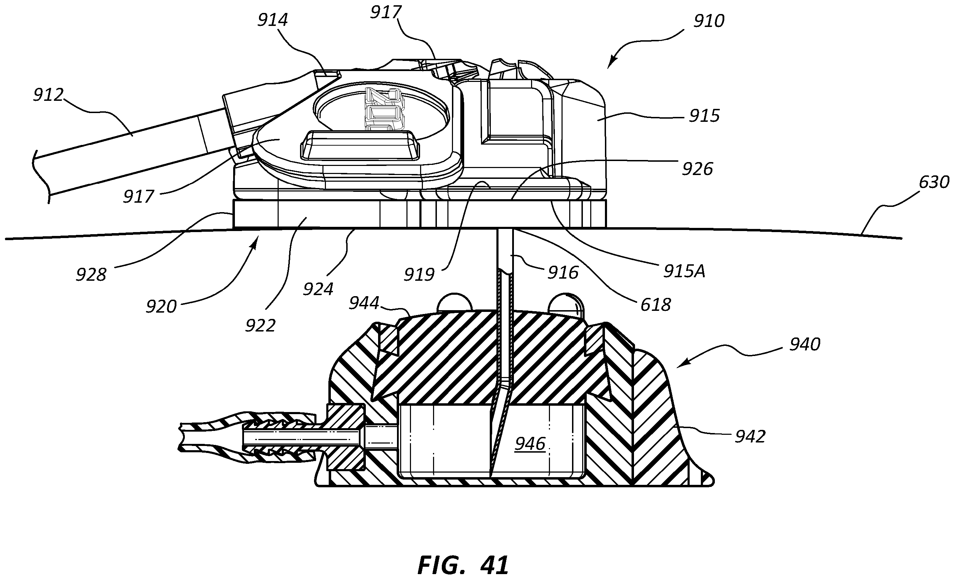

As seen in FIG. 41, the needle assembly 910 of the infusion set 908 can be employed to access a subcutaneously implanted access port 940. As shown, the access port 940 includes a body 942 and a septum 944 that cooperate to define a reservoir 946.

As has been described, the contact surface 924 of the pad 920 of the infusion set needle assembly 910 rests against the skin 630 when the needle 916 of the needle assembly 910 is percutaneously disposed within the patient, such as in accessing the access port 940 shown in FIG. 41. This enables the antimicrobial/haemostatic agents included with the interface pad 920 to be in contact with and treat/protect the needle insertion site 618.

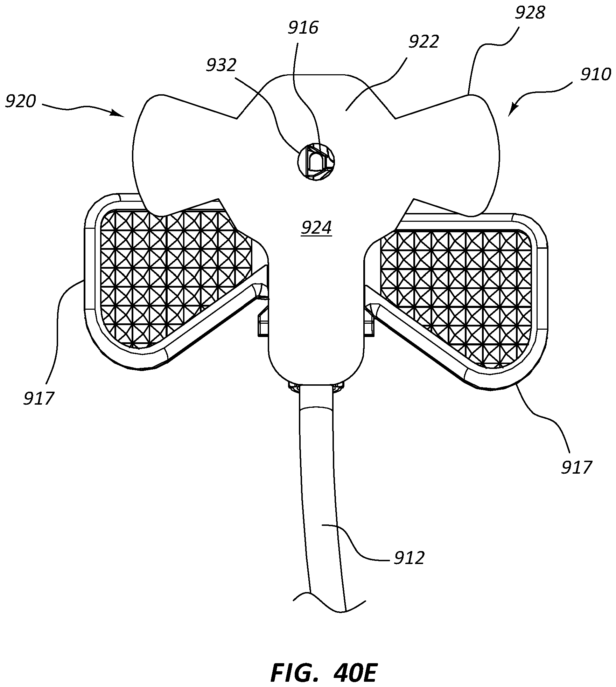

In another embodiment, it is appreciated that the hole of the interface pad, such as the hole 932 shown in FIG. 40E, can be sized larger than what is required for passage of the needle 916. Note that the shape of the interface pad can be formed a number of ways, including die-cut. Thus, it is appreciated that the interface pad can include one of a variety of shapes, sizes, and configurations, and can be employed on a variety of percutaneous medical devices. In one example, for example, the size of the interface pad is selected so as to not obscure the view of the clinician placing the medical device into the patient.

## Claims

1. A needle assembly (810, 910), comprising:
a needle hub (814, 914); and
a needle (816, 916) extending from the needle hub (814, 914); and
an interface pad (820, 920) attached to the needle hub (814, 914) before use of the needle assembly (810, 910), the interface pad (820, 920) including at least one of a haemostatic agent and an antimicrobial agent, the interface pad (820, 920) configured to be interposed between the needle hub (814, 914) and the patient's skin when the needle (816, 916) is percutaneously inserted into the patient,
**characterized in that**
the interface pad (820, 920) includes shape that matches a shape of a bottom surface (815A, 915A) of the needle hub (814, 914), the interface pad (820, 920) including a hole through which the needle (816, 916) is configured to extend.

2. The needle assembly (810, 910) as defined in claim 1, wherein the interface pad (820, 920) is configured to cover at least a portion of an insertion site through which the needle (816, 916) penetrates the skin of the patient such that the at least one of the haemostatic agent and the antimicrobial agent contacts the insertion site.

3. The needle assembly (810, 910) as defined in claim 1 or 2, wherein the interface pad (820, 920) is permanently attached to the needle hub (814, 914), or wherein the interface pad (820, 920) is temporarily attached to the needle hub (814, 914), the interface pad (820, 920) including a slit to enable removal from the needle hub (814, 914).

4. The needle assembly (810, 910) as defined in any one of the preceding claims, wherein the needle assembly (810, 910) is included as part of an infusion set for accessing an implanted access port.

5. The needle assembly (810, 910) as defined in any one of the preceding claims, wherein the needle hub (814, 914) includes an extensible base (815, 915), the interface pad (820, 920) attached to the base (815, 915).

6. The needle assembly (810, 910) as defined in any one of the preceding claims, wherein the antimicrobial agent includes chlorhexidine gluconate.

7. The needle assembly (810, 910) as defined in any one of the preceding claims, wherein the haemostatic agent includes a hydrocolloid and/or wherein the haemostatic agent include microdispersed oxidized cellulose.

8. The needle assembly (810, 910) as defined in any one of the preceding claims, wherein the interface pad (820, 920) includes a compliant material to provide patient comfort when the needle assembly (810, 910) is disposed on the skin of the patient.

9. The needle assembly (810, 910) as defined in claim 8, wherein the interface pad (820, 920) includes a foam material, wherein preferably the foam material includes an aromatic polyether polyurethane.

10. The needle assembly (810, 910) as defined in any one of the preceding claims, wherein the interface pad (820, 920) includes an absorptive material and wherein the interface pad (820, 920) is sized so as to not obscure the view of a clinician inserting the needle assembly (810, 910) into the patient.

11. A method of manufacturing a needle assembly (810, 910), the method comprising: providing a needle hub (814, 914), a needle (816, 916) distally extending from the needle hub (814, 914); and permanently adhering an interface pad (820, 920) to a bottom surface (815A, 915A) of the needle hub (814, 914),
the needle (816, 916) configured to extend through the interface pad (820, 920), the interface pad (820, 920) including at least one of a haemostatic agent and an antimicrobial agent.

12. The method of manufacturing as defined in claim 11, wherein providing the needle hub (814, 914) includes providing the needle hub (814, 914) with an extensible base (815, 915), the interface pad (820, 920) permanently adhered to the bottom surface (815A, 915A) of the base (815, 915) of the needle hub (814, 914).

13. The method of manufacturing as defined in claim 11 or 12, wherein permanently adhering includes adhering the interface pad (820, 920) such that the needle (816, 916) of the needle hub (814, 914) extends through a hole defined in the interface pad (820, 920).

## Patentansprüche

1. Nadelanordnung (810, 910), umfassend:
eine Nadelansatz (814, 914); und
eine Nadel (816, 916), die sich von dem Nadelansatz (814, 914) erstreckt; und
einen Schnittstellenpfad (820, 920), der an dem Nadelansatz (814, 914) vor Verwendung der Nadelanordnung (810, 910) befestigt wird, wobei der Schnittstellenpfad (820, 920) mindestens eines von einem Hämostatikum und einem Antimikrobiotikum einschließt, wobei der Schnittstellenpfad (820, 920) so konfiguriert ist, dass er zwischen dem Nadelansatz (814, 914) und der Haut des Patienten zwischengesetzt ist, wenn die Nadel (816, 916) perkutan in den Patienten eingeführt ist,
**dadurch gekennzeichnet, dass**
der Schnittstellenpfad (820, 920) eine Form einschließt, die zu einer Form einer unteren Fläche (815A, 915A) des Nadelansatzes (814, 914) passt, wobei der Schnittstellenpfad (820, 920) ein Loch einschließt, wobei die Nadel (816, 916) so konfiguriert ist, dass sie sich dadurch erstreckt.

2. Nadelanordnung (810, 910) nach Anspruch 1, wobei der Schnittstellenpfad (820, 920) so konfiguriert ist, dass er mindestens einen Abschnitt einer Einführungsstelle abdeckt, durch die die Nadel (816, 916) die Haut des Patienten so durchdringt, dass das mindestens eine des Hämostatikums und des Antimikrobiotikums mit der Einführungsstelle in Berührung ist.

3. Nadelanordnung (810, 910) nach Anspruch 1 oder 2, wobei der Schnittstellenpfad (820, 920) permanent an dem Nadelansatz (814, 914) befestigt ist oder wobei der Schnittstellenpfad (820, 920) vorübergehend an dem Nadelansatz (814, 914) befestigt ist, wobei der Schnittstellenpfad (820, 920) einen Schlitz einschließt, um das Entnehmen von dem Nadelansatz (814, 914) zu ermöglichen.

4. Nadelanordnung (810, 910) nach einem der vorstehenden Ansprüche, wobei die Nadelanordnung (810, 910) als Teil eines Infusionssets für Zugang zu einem implantierten Zugangsanschluss eingeschlossen ist.

5. Nadelanordnung (810, 910) nach einem der vorstehenden Ansprüche, wobei der Nadelansatz (814, 914) eine extensible Basis (815, 915) einschließt, wobei der Schnittstellenpfad (820, 920) an der Basis (815, 915) befestigt ist.

6. Nadelanordnung (810, 910) nach einem der vorstehenden Ansprüche, wobei das Antimikrobiotikum Chlorhexidingluconat einschließt.

7. Nadelanordnung (810, 910) nach einem der vorstehenden Ansprüche, wobei das Hämostatikum ein Hydrokolloid einschließt und/oder wobei das Hämostatikum hochdispergierte oxidierte Zellulose einschließt.

8. Nadelanordnung (810, 910) nach einem der vorstehenden Ansprüche, wobei der Schnittstellenpfad (820, 920) ein elastisches Material einschließt, um Patientenkomfort bereitzustellen, wenn die Nadelanordnung (810, 910) auf der Haut des Patienten angeordnet ist.

9. Nadelanordnung (810, 910) nach Anspruch 8, wobei der Schnittstellenpfad (820, 920) ein Schaummaterial einschließt, wobei das Schaummaterial bevorzugt ein aromatisches Polyetherpolyurethan einschließt.

10. Nadelanordnung (810, 910) nach einem der vorstehenden Ansprüche, wobei der Schnittstellenpfad (820, 920) ein absorbierendes Material einschließt und wobei der Schnittstellenpfad (820, 920) so bemessen ist, dass er die Sicht eines Klinikers, der die Nadelanordnung (810, 910) in den Patienten einführt, nicht verdeckt.

11. Verfahren zum Herstellen einer Nadelanordnung (810, 910), wobei das Verfahren Folgendes umfasst: Bereitstellen eines Nadelansatzes (814, 914), einer Nadel (816, 916), die sich von dem Nadelansatz (814, 914) distal erstreckt; und permanentes Anheften eines Schnittstellenpfads (820, 920) an eine untere Fläche (815A, 915A) des Nadelansatzes (814, 914),
wobei die Nadel (816, 916) so konfiguriert ist, dass sie sich durch den Schnittstellenpfad (820, 920) erstreckt, wobei der Schnittstellenpfad (820, 920) mindestens eines von einem Hämostatikum und einem Antimikrobiotikum einschließt.

12. Verfahren zum Herstellen nach Anspruch 11, wobei das Bereitstellen des Nadelansatzes (814, 914) das Bereitstellen des Nadelansatzes (814, 914) mit einer extensiblen Basis (815, 915) einschließt, wobei der Schnittstellenpfad (820, 920) permanent an die untere Fläche (815A, 915A) der Basis (815, 915) des Nadelansatzes (814, 914) angeheftet ist.

13. Verfahren zum Herstellen nach Anspruch 11 oder 12, wobei das permanente Anheften das Anheften des Schnittstellenpfads (820, 920) so einschließt, dass sich die Nadel (816, 916) von dem Nadelansatz (814, 914) durch ein Loch erstreckt, das im Schnittstellenpfad (820, 920) definiert ist.

## Revendications

1. Ensemble aiguille (810, 910), comprenant :
un pavillon d'aiguille (814, 914) ; et
une aiguille (816, 916) s'étendant à partir du pavillon d'aiguille (814, 914) ; et
un tampon d'interface (820, 920) fixé au pavillon d'aiguille (814, 914) avant une utilisation de l'ensemble aiguille (810, 910), le tampon d'interface (820, 920) incluant au moins un agent parmi un agent homéostatique et un agent antimicrobien, le tampon d'interface (820, 920) étant configuré pour être intercalé entre le pavillon d'aiguille (814, 914) et la peau du patient lorsque l'aiguille (816, 916) est insérée par voie percutanée dans le patient,
**caractérisé en ce que**
le tampon d'interface (820, 920) inclut une forme qui correspond à une forme d'une surface inférieure (815A, 915A) du pavillon d'aiguille (814, 914), le tampon d'interface (820, 920) incluant un trou à travers lequel l'aiguille (816, 916) est configurée pour s'étendre.

2. Ensemble aiguille (810, 910) tel que défini dans la revendication 1, dans lequel le tampon d'interface (820, 920) est configuré pour recouvrir au moins une partie d'un site d'insertion à travers lequel l'aiguille (816, 916) pénètre dans la peau du patient de sorte que l'au moins un agent parmi l'agent homéostatique et l'agent antimicrobien vienne au contact du site d'insertion.

3. Ensemble aiguille (810, 910) tel que défini dans la revendication 1 ou 2, dans lequel le tampon d'interface (820, 920) est fixé de manière permanente au pavillon d'aiguille (814, 914), ou dans lequel le tampon d'interface (820, 920) est fixé de manière temporaire au pavillon d'aiguille (814, 914), le tampon d'interface (820, 920) incluant une fente de manière à permettre un retrait à partir du pavillon d'aiguille (814, 914).

4. Ensemble aiguille (810, 910) tel que défini dans l'une quelconque des revendications précédentes, dans lequel l'ensemble aiguille (810, 910) est inclus en tant que partie d'un ensemble de perfusion permettant d'accéder à un port d'accès implanté.

5. Ensemble aiguille (810, 910) tel que défini dans l'une quelconque des revendications précédentes, dans lequel le pavillon d'aiguille (814, 914) inclut une base extensible (815, 915), le tampon d'interface (820, 920) étant fixé à la base (815, 915).

6. Ensemble aiguille (810, 910) tel que défini dans l'une quelconque des revendications précédentes, dans lequel l'agent antimicrobien inclut du gluconate de chlorhexidine.

7. Ensemble aiguille (810, 910) tel que défini dans l'une quelconque des revendications précédentes, dans lequel l'agent homéostatique inclut un hydrocolloïde et/ou dans lequel l'agent homéostatique inclut une cellulose oxydée microdispersée.

8. Ensemble aiguille (810, 910) tel que défini dans l'une quelconque des revendications précédentes, dans lequel le tampon d'interface (820, 920) inclut un matériau élastique pour apporter un confort au patient lorsque l'ensemble aiguille (810, 910) est disposé sur la peau du patient.

9. Ensemble aiguille (810, 910) tel que défini dans la revendication 8, dans lequel le tampon d'interface (820, 920) inclut un matériau en mousse, dans lequel, de préférence, le matériau en mousse inclut un polyuréthane de polyéther aromatique.

10. Ensemble aiguille (810, 910) tel que défini dans l'une quelconque des revendications précédentes, dans lequel le tampon d'interface (820, 920) inclut un matériau absorbant et dans lequel le tampon d'interface (820, 920) est dimensionné de manière à ne pas cacher la vue d'un clinicien insérant l'ensemble aiguille (810, 910) dans le patient.

11. Procédé de fabrication d'un ensemble aiguille (810, 910), le procédé comprenant : une fourniture d'un pavillon d'aiguille (814, 914), une aiguille (816, 916) s'étendant distalement à partir du pavillon d'aiguille (814, 914) ; et un collage de manière permanente d'un tampon d'interface (820, 920) à une surface inférieure (815A, 915A) du pavillon d'aiguille (814, 914),
l'aiguille (816, 916) étant configurée pour s'étendre à travers le tampon d'interface (820, 920), le tampon d'interface (820, 920) incluant au moins un agent parmi un agent homéostatique et un agent antimicrobien.

12. Procédé de fabrication tel que défini dans la revendication 11, dans lequel la fourniture du pavillon d'aiguille (814, 914) inclut une fourniture du pavillon d'aiguille (814, 914) avec une base extensible (815, 915), le tampon d'interface (820, 920) étant collé de manière permanente à la surface inférieure (815A, 915A) de la base (815, 915) du pavillon d'aiguille (814, 914).

13. Procédé de fabrication tel que défini dans la revendication 11 ou 12, dans lequel le collage de manière permanente inclut un collage du tampon d'interface (820, 920) de sorte que l'aiguille (816, 916) du pavillon d'aiguille (814, 914) s'étende à travers un trou défini dans le tampon d'interface (820, 920).
